# EUROPEAN PATENT SPECIFICATION

(11) **EP 4 051 080 B1**
(45) Date of publication and mention of the grant of the patent: **19.02.2025**
(21) Application number: 20828152.7
(22) Date of filing: 10.12.2020
(51) Int. Cl.: A61B 1/045, A61B 34/37, A61B 34/00, A61B 34/20, A61B 34/30, A61B 90/00, A61B 1/00, A61B 17/00, A61B 90/30

(54) **METHOD, APPARATUS AND SYSTEM FOR CONTROLLING AN IMAGE CAPTURE DEVICE DURING SURGERY**
VERFAHREN, VORRICHTUNG UND SYSTEM ZUM STEUERN EINER BILDAUFNAHMEVORRICHTUNG WÄHREND EINER OPERATION
PROCÉDÉ, APPAREIL ET SYSTÈME DE COMMANDE D'UN DISPOSITIF DE CAPTURE D'IMAGE PENDANT UNE CHIRURGIE

(30) Priority: 19.12.2019 EP 19218155
(43) Date of publication of application: 07.09.2022
(73) Proprietor: Sony Group Corporation, Tokyo 108-0075 (JP)
(72) Inventor: WRIGHT, Christopher, London SE193HF (GB); ELLIOTT-BOWMAN, Bernadette, London SE193HF (GB); KAMODA, Akinori, Tokyo 108-0075 (JP); KURODA, Yohei, Tokyo 108-0075 (JP)
(74) Representative: D Young & Co LLP
(86) International application number: PCT/JP2020/046146
(87) International publication number: WO 2021/125056

(56) References cited:
- EP-A1- 3 501 443
- US-A1- 2014 051 921

## Description

### Field

The present disclosure relates to a method, program and system for controlling an image capture device during surgery.

### Background

The "background" description provided herein is for the purpose of generally presenting the context of the disclosure. Work of the presently named inventors, to the extent it is described in the background section, as well as aspects of the description which may not otherwise qualify as prior art at the time of filing, are neither expressly or impliedly admitted as prior art against the present disclosure.

In recent years, significant technological developments in medical systems and equipment have been achieved. Computer assisted surgical systems, such as robotic systems, now often work alongside a human surgeon during surgery. These computer assisted surgery systems include master-slave type robotic systems in which a human surgeon operates a master console in order to control the operations of a slave device during surgery.

Computer assisted camera systems, such as robotic camera systems, are used in a surgical environment to provide visual information to a human operator or surgeon. These computer assisted camera systems may be equipped with a single camera capturing and providing a view of surgical action within the scene. Alternatively, these computer assisted camera systems may include a plurality of cameras which each capture a given view of the surgical action within the scene.

However, the view onto a surgical scene often becomes obstructed by elements such as tools, the surgeon's hands, tissue deformations or other dynamic elements. That is, surgical scenes are often very complex and include many dynamic elements which may obstruct the view obtained from a medical image capture device such as an endoscope or a microscope.

For example, EP 3 501 443 discloses a system for controlling medical cameras during surgery. The cameras track an object within a surgical field and the system predicts, based on the acquired tracking images, if the field of view of a given camera will get obstructed within few image frames. If this is the case, said camera is rotated to avoid the obstruction so that subsequent tracking images can be acquired.

These obstructions can delay the capture and provision of critical visual information which is required in order to inform a surgeon or robot system of a change in the scene.

In a surgical environment, the delay of providing critical visual information to the surgeon can have significant consequences.

It is an aim of the present disclosure to address these issues.

### Summary

According to a first aspect of the present disclosure, a system for controlling a medical image capture device during surgery is provided, the system including circuitry configured to: acquire first image data from the medical image capture device, the first image data being of an appearance of a surgical scene at a first instance of time; determine, based on a predicted appearance of the surgical scene based on the first image data at a second instance of time after the first instance of time, one or more desired image capture properties of the medical image capture device; and control the medical image capture device at a third instance of time, the third instance of time being between the first instance of time and the second instance of time, in accordance with the one or more desired image capture properties of the medical image capture device. Further, the circuitry is configured to determine a desired imaging condition of the image capture device as one of the one or more desired image capture properties of the medical image capture device; wherein the desired imaging condition includes one or more of an optical image system condition and an image processing condition and wherein the optical image system condition and the image processing condition include at least one of an image zoom, an image focus, an image aperture, an image contrast and/or an image brightness of the medical image capture device.

According to a second aspect of the present disclosure, a method of controlling a medical image capture device during surgery is provided, the method including: acquiring first image data from the medical image capture device, the first image data being of an appearance of a surgical scene at a first instance of time; determining, based on a predicted appearance of the surgical scene based on the first image data at a second instance of time after the first instance of time, one or more desired image capture properties of the medical image capture device; and controlling the medical image capture device at a third instance of time, the third instance of time being between the first instance of time and the second instance of time, in accordance with the one or more desired image capture properties of the medical image capture device.

Further, the method encompasses determination of a desired imaging condition of the image capture device as one of the one or more desired image capture properties of the medical image capture device; wherein the desired imaging condition includes one or more of an optical image system condition and an image processing condition and wherein the optical image system condition and the image processing condition include at least one of an image zoom, an image focus, an image aperture, an image contrast and/or an image brightness of the medical image capture device.

According to a third aspect of the present disclosure, a computer program product including instructions which, when the program is executed by a computer, cause the computer to carry out a method of controlling a medical image capture device is provided, the method including: acquiring first image data from the medical image capture device, the first image data being of an appearance of a surgical scene at a first instance of time; determining, based on a predicted appearance of the surgical scene based on the first image data at a second instance of time after the first instance of time, one or more desired image capture properties of the medical image capture device; and controlling the medical image capture device at a third instance of time, the third instance of time being between the first instance of time and the second instance of time, in accordance with the one or more desired image capture properties of the medical image capture device. Further, the program is configured to determine a desired imaging condition of the image capture device as one of the one or more desired image capture properties of the medical image capture device; wherein the desired imaging condition includes one or more of an optical image system condition and an image processing condition and wherein the optical image system condition and the image processing condition include at least one of an image zoom, an image focus, an image aperture, an image contrast and/or an image brightness of the medical image capture device.

Aspects of the present disclosure enable a computer assisted camera system to predictively optimise the position of the camera, such that impact of predictable future changes to the surgical utility and predictability of a captured scene are accounted for through movements of the camera system before the change occurs. This reduces the delay in the capture and provision of critical visual information to the surgeon or surgical robotic system during surgery.

The invention is defined in the following claims. Other embodiments, examples, method steps etc. are not a part of the invention. The described embodiments, together with further advantages, will be best understood by reference to the following detailed description taken in conjunction with the accompanying drawings.

### Brief Description of Drawings

A more complete appreciation of the disclosure and many of the attendant advantages thereof will be readily obtained as the same becomes better understood by reference to the following detailed description when considered in connection with the accompanying drawings.
[fig.1]Fig. 1 is a diagram illustrating an example of a schematic configuration of an endoscopic surgery system to which a medical support arm device according to the present disclosure can be applied.
[fig.2]Fig. 2 is a block diagram illustrating an example of functional configurations of a camera head and a CCU (camera control unit) illustrated in Fig. 1.
[fig.3]Fig. 3 is an explanatory diagram illustrating a use example master apparatus according to the present disclosure.
[fig.4]Fig. 4 shows an apparatus for controlling an image capture device during surgery in accordance with embodiments of the disclosure.
[fig.5A]Fig. 5A illustrates an example situation to which embodiments of the disclosure may be applied.
[fig.5B]Fig. 5B illustrates an example first image data in accordance with embodiments of the disclosure.
[fig.5C]Fig. 5C illustrates a time chart of the generation of the predicted appearance of a surgical scene in accordance with embodiments of the disclosure.
[fig.5D]Fig. 5D illustrates an example second image data in accordance with embodiments of the disclosure.
[fig.5E]Fig. 5E illustrates an example image obtained by an image capture device during surgery in accordance with embodiments of the disclosure.
[fig.6A]Fig. 6A illustrates an apparatus for controlling an image capture device during surgery in accordance with embodiments of the disclosure.
[fig.6B]Fig. 6B illustrates an example situation to which embodiments of the disclosure may be applied.
[fig.7A]Fig. 7A illustrates an example setup of a computer assisted surgical system in accordance with embodiments of the present.
[fig.7B]Fig. 7B illustrates an example situation to which embodiments of the disclosure may be applied.
[fig.7C]Fig. 7C illustrates an example situation to which embodiments of the disclosure may be applied.
[fig.7D]Fig. 7D illustrates an example situation to which embodiments of the disclosure may be applied.
[fig.7E]Fig. 7E illustrates an example situation to which embodiments of the disclosure may be applied.
[fig.8]Fig. 8 illustrates a method of controlling an image capture device during surgery in accordance with embodiments of the disclosure.
[fig.9]Fig. 9 shows a computing device for controlling an image capture device during surgery in accordance with embodiments of the disclosure.
[fig.10]Fig. 10 schematically shows a first example of a computer assisted surgery system to which the present technique is applicable.
[fig.11] Fig. 11 schematically shows a second example of a computer assisted surgery system to which the present technique is applicable.
[fig.12]Fig. 12 schematically shows a third example of a computer assisted surgery system to which the present technique is applicable.
[fig.13]Fig. 13 schematically shows a fourth example of a computer assisted surgery system to which the present technique is applicable.
[fig.14]Fig. 14 schematically shows an example of an arm unit.

### Description of Embodiments

Referring now to the drawings, wherein like reference numerals designate identical or corresponding parts throughout the several views.

### <<1. Basic Configuration>>

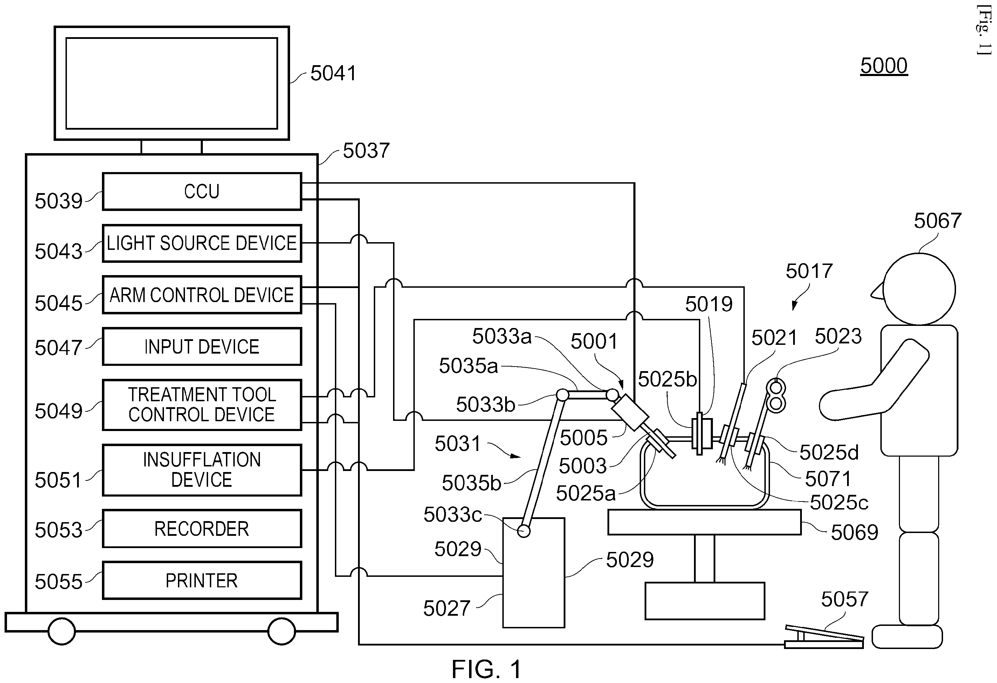

First, a basic configuration of an endoscopic surgery system to which embodiments of the disclosure may be applied will be described with reference to Figs. 1 to 4 of the present disclosure.

### <1.1. Configuration Example of Endoscopic Surgery System>

Fig. 1 is a diagram illustrating an example of a schematic configuration of an endoscopic surgery system 5000 to which the technology according to the present disclosure can be applied. Fig. 1 illustrates a state where an operator (doctor) 5067 is conducting surgery to a patient 5071 on a patient bed 5069 using the endoscopic surgery system 5000. As illustrated, the endoscopic surgery system 5000 is constituted by an endoscope 5001, other surgical tools 5017, and a support arm device 5027 supporting the endoscope 5001, and a cart 5037 on which various devices for endoscopic surgery are mounted.

In the endoscopic surgery, the abdominal wall is punctured with a plurality of tubular hole-opening instruments called trocars 5025a to 5025d instead of cutting the abdominal wall to open the abdomen. Then, a lens barrel 5003 of the endoscope 5001 and the other surgical tools 5017 are inserted into a body cavity of the patient 5071 through the trocars 5025a to 5025d. In the illustrated example, as the other surgical tools 5017, an insufflation tube 5019, an energy treatment tool 5021, and forceps 5023 are inserted into the body cavity of the patient 5071. Furthermore, the energy treatment tool 5021 is a treatment tool that performs incision and peeling of a tissue, sealing of a blood vessel, or the like using high-frequency current or ultrasonic vibration. However, the illustrated surgical tool 5017 is merely an example, and various surgical tools generally used in endoscopic surgery, for example, tweezers, a retractor, and the like may be used as the surgical tool 5017.

An image of an operation site in the body cavity of the patient 5071 captured by the endoscope 5001 is displayed on a display device 5041. The operator 5067 performs treatment, for example, to excise an affected site using the energy treatment tool 5021 or the forceps 5023 while viewing the image of the operation site displayed by the display device 5041 in real time. Note that the insufflation tube 5019, the energy treatment tool 5021, and the forceps 5023 are supported by the operator 5067, an assistant, or the like during surgery although not illustrated.

### (Support Arm Device)

The support arm device 5027 includes an arm unit 5031 extending from a base unit 5029. In the illustrated example, the arm unit 5031 is a multi-joint arm constituted by joints 5033a, 5033b, and 5033c and links 5035a and 5035b, and is driven by control from an arm control device 5045. The arm unit 5031 has a distal end to which the endoscope 5001 can be connected. The endoscope 5001 is supported by the arm unit 5031, and a position and a posture thereof are controlled. With the configuration, it is possible to realize stable fixing of the position of the endoscope 5001.

### (Endoscope)

The endoscope 5001 is constituted by the lens barrel 5003 having a region of a predetermined length from a distal end that is inserted into the body cavity of the patient 5071, and a camera head 5005 connected to a proximal end of the lens barrel 5003. Although the endoscope 5001 configured as a so-called rigid scope having the rigid lens barrel 5003 is illustrated in the illustrated example, the endoscope 5001 may be configured as a so-called flexible scope having the flexible lens barrel 5003.

An opening portion into which an objective lens is fitted is provided at the distal end of the lens barrel 5003. A light source device 5043 is connected to the endoscope 5001, and light generated by the light source device 5043 is guided to the distal end of the lens barrel by a light guide extended inside the lens barrel 5003 and is emitted toward an observation object in the body cavity of the patient 5071 through the objective lens. Note that the endoscope 5001 may be a forward-viewing scope, an oblique-viewing scope, or a side-viewing scope.

An optical system and an imaging element are provided inside the camera head 5005, and reflected light (observation light) from the observation object is collected on the imaging element by the optical system. The observation light is photoelectrically converted by the imaging element, and an electric signal corresponding to the observation light, in other words, an image signal corresponding to an observation image is generated. The image signal is transmitted as RAW data to a camera control unit (CCU) 5039. Note that the camera head 5005 is equipped with a function of adjusting magnification and a focal length by properly driving the optical system.

Note that a plurality of imaging elements may be provided in the camera head 5005, for example, in order to cope with stereoscopic viewing (3D display) or the like. In this case, a plurality of relay optical systems is provided inside the lens barrel 5003 in order to guide the observation light to each of the plurality of imaging elements.

### (Various Devices Equipped in Cart)

The CCU 5039 is configured using a central processing unit (CPU), a graphics processing unit (GPU), or the like, and integrally controls operations of the endoscope 5001 and the display device 5041. Specifically, the CCU 5039 performs various types of image processing, for example, development processing (demosaicing processing) or the like on an image signal received from the camera head 5005 to display an image based on the image signal. The CCU 5039 provides the image signal subjected to the image processing to the display device 5041. Furthermore, the CCU 5039 transmits a control signal to the camera head 5005 and controls drive of the camera head 5005. The control signal may include information regarding imaging conditions such as magnification and a focal length.

The display device 5041 displays an image based on the image signal subjected to image processing by the CCU 5039 under the control of the CCU 5039. In a case where the endoscope 5001 is an endoscope compatible with high-resolution capturing, for example, 4K (the number of horizontal pixels of 3840 × the number of vertical pixels of 2160), 8K (the number of horizontal pixels of 7680 × the number of vertical pixels of 4320) or the like, and/or in a case of an endoscope compatible with 3D display, a device capable of high-resolution display and/or a device capable of 3D display can be used as the display device 5041 to be compatible with the above endoscopes, respectively. In the case of the endoscope compatible with the high-resolution capturing such as 4K and 8K, a more immersive feeling can be obtained by using the display device 5041 having a size of 55 inches or more. Furthermore, a plurality of the display devices 5041 having different resolutions and sizes may be provided in accordance with an application.

The light source device 5043 is configured using a light source such as a light emitting diode (LED), for example, and supplies irradiation light at the time of capturing an operation site to the endoscope 5001.

The arm control device 5045 is configured using a processor, for example, a CPU or the like, and operates according to a predetermined program to control the drive of the arm unit 5031 of the support arm device 5027 according to a predetermined control method.

The input device 5047 is an input interface with respect to the endoscopic surgery system 5000. A user can input various types of information and instructions to the endoscopic surgery system 5000 via the input device 5047. For example, the user inputs various types of information regarding surgery, such as information regarding a patient's body and information regarding surgical operation technology via the input device 5047. Furthermore, for example, the user inputs an instruction to drive the arm unit 5031, an instruction to change an imaging condition (a type of irradiated light, magnification, a focal length, or the like) using the endoscope 5001, an instruction to drive the energy treatment tool 5021, and the like via the input device 5047.

The type of the input device 5047 is not limited, and the input device 5047 may be various known input devices. For example, a mouse, a keyboard, a touch panel, a switch, a foot switch 5057 and/or a lever can be applied as the input device 5047. In a case where a touch panel is used as the input device 5047, the touch panel may be provided on a display surface of the display device 5041.

Alternatively, the input device 5047 is, for example, a device to be mounted by the user, such as a glasses-type wearable device and a head-mounted display (HMD), and various inputs are performed in accordance with a gesture or a line of sight of the user detected by these devices. Furthermore, the input device 5047 includes a camera capable of detecting user's motion, and various inputs are performed in accordance with a gesture or a line of sight of the user detected from an image captured by the camera. Moreover, the input device 5047 includes a microphone capable of collecting user's voice, and various inputs are performed using the voice through the microphone. In this manner, the input device 5047 is configured to be capable of inputting various types of information in a non-contact manner, and particularly, the user (for example, the operator 5067) belonging to a clean area can operate equipment belonging to an unclean area in a non-contact manner. Furthermore, the user can operate the equipment without releasing his/her hand from the possessed surgical tool, and thus, the convenience of the user is improved.

The treatment tool control device 5049 controls the drive of the energy treatment tool 5021 for cauterization of a tissue, an incision, sealing of a blood vessel, or the like. An insufflation device 5051 sends a gas into a body cavity through the insufflation tube 5019 in order t to inflate the body cavity of the patient 5071 for the purpose of securing a visual field by the endoscope 5001 and securing a working space for the operator. A recorder 5053 is a device capable of recording various types of information regarding surgery. A printer 5055 is a device capable of printing various types of information regarding surgery in various formats such as text, an image, and a graph.

Hereinafter, a particularly characteristic configuration in the endoscopic surgery system 5000 will be described in more detail.

### (Support Arm Device)

The support arm device 5027 includes the base unit 5029 as a base and the arm unit 5031 extending from the base unit 5029. Although the arm unit 5031 is constituted by the plurality of joints 5033a, 5033b, and 5033c, and the plurality of links 5035a and 5035b connected by the joint 5033b in the illustrated example, Fig. 1 illustrates the configuration of the arm unit 5031 in a simplified manner for the sake of simplicity. Actually, each shape, the number, and the arrangement of the joints 5033a to 5033c and the links 5035a and 5035b, a direction of a rotation axis of each of the joints 5033a to 5033c, and the like are appropriately set such that the arm unit 5031 has a desired degree of freedom. For example, the arm unit 5031 can be preferably configured to have the degree of freedom equal to or greater than six degrees of freedom. With the configuration, the endoscope 5001 can be freely moved within a movable range of the arm unit 5031, and thus, it is possible to insert the lens barrel 5003 of the endoscope 5001 into the body cavity of the patient 5071 from a desired direction.

Actuators are provided in the joints 5033a to 5033c, and the joints 5033a to 5033c are configured to be rotatable about a predetermined rotation axis by the drive of the actuators. As the drive of the actuator is controlled by the arm control device 5045, each rotation angle of the joints 5033a to 5033c is controlled, and the drive of the arm unit 5031 is controlled. With the configuration, the control of the position and the posture of the endoscope 5001 can be realized. At this time, the arm control device 5045 can control the drive of the arm unit 5031 by various known control methods such as force control or position control.

For example, the position and posture of the endoscope 5001 may be controlled as the operator 5067 appropriately performs an operation input via the input device 5047 (including the foot switch 5057) and the drive of the arm unit 5031 is appropriately controlled by the arm control device 5045 according to the operation input. Through such control, the endoscope 5001 at the distal end of the arm unit 5031 can be moved from an arbitrary position to an arbitrary position, and then, fixedly supported at a position after the movement. Note that the arm unit 5031 may be operated in a so-called master-slave manner. In this case, the arm unit 5031 can be remotely operated by the user via the input device 5047 installed at a place distant from an operating room.

Furthermore, in a case where the force control is applied, the arm control device 5045 may receive an external force from the user and perform so-called power assist control to drive the actuators of the joints 5033a to 5033c such that the arm unit 5031 moves smoothly according to the external force. With the configuration, when the user moves the arm unit 5031 while directly touching the arm unit 5031, the arm unit 5031 can be moved with a relatively light force. Therefore, it is possible to more intuitively move the endoscope 5001 with a simpler operation, and it is possible to improve the convenience of the user.

Here, the endoscope 5001 has been generally supported by a doctor called a scopist in endoscopic surgery. In regard to this, it becomes possible to more reliably fix the position of the endoscope 5001 without human hands by using the support arm device 5027, and thus, it is possible to stably obtain an image of an operation site and to smoothly perform the surgery.

Note that the arm control device 5045 is not necessarily provided in the cart 5037. Furthermore, the arm control device 5045 is not necessarily one device. For example, the arm control device 5045 may be provided at each of joints 5033a to 5033c of the arm unit 5031 of the support arm device 5027, or the drive control of the arm unit 5031 may be realized by the plurality of arm control devices 5045 cooperating with each other.

### (Light Source Device)

The light source device 5043 supplies irradiation light at the time of capturing an operation site to the endoscope 5001. The light source device 5043 is configured using, for example, a white light source constituted by an LED, a laser light source, or a combination thereof. At this time, in a case where the white light source is constituted by a combination of RGB laser light sources, the output intensity and output timing of each color (each wavelength) can be controlled with high precision, and thus, it is possible to adjust white balance of a captured image in the light source device 5043. Furthermore, in this case, it is also possible to capture an image corresponding to each of RGB in a time-division manner by irradiating an observation object with laser light from each of the RGB laser light sources in a time-division manner and controlling the drive of the imaging element of the camera head 5005 in synchronization with an irradiation timing. According to this method, a color image can be obtained without providing a color filter in the imaging element.

Furthermore, the drive of the light source device 5043 may be controlled so as to change the intensity of light to be output every predetermined time. The drive of the imaging element of the camera head 5005 is controlled in synchronization with a timing of the change of the light intensity to acquire images in a time-division manner, and a so-called high dynamic range image without so-called crushed blacks and blown-out whites can be generated by combining the images.

Furthermore, the light source device 5043 may be configured to be capable of supplying light in a predetermined wavelength band which is compatible with special light observation. In the special light observation, for example, the wavelength dependency of light absorption in a body tissue is utilized, and light is emitted in a narrow band as compared to irradiation light during normal observation (in other words, white light), thereby performing so-called narrow band imaging (NBI) in which a predetermined tissue, such as a blood vessel in a superficial portion of a mucous membrane, is captured at a high contrast. Alternatively, fluorescent observation that obtains an image with fluorescent light generated by emitting excitation light may also be performed in the special light observation. In the fluorescence observation, it is possible to irradiate a body tissue with excitation light and observe fluorescent light from the body tissue (autofluorescence observation), to locally inject a reagent such as indocyanine green (ICG) into a body tissue and also irradiate the body tissue with excitation light corresponding to a fluorescence wavelength of the reagent to obtain a fluorescent image, or the like. The light source device 5043 can be configured to be capable of supplying narrow-band light and/or excitation light corresponding to such special light observation.

### (Camera Head and CCU)

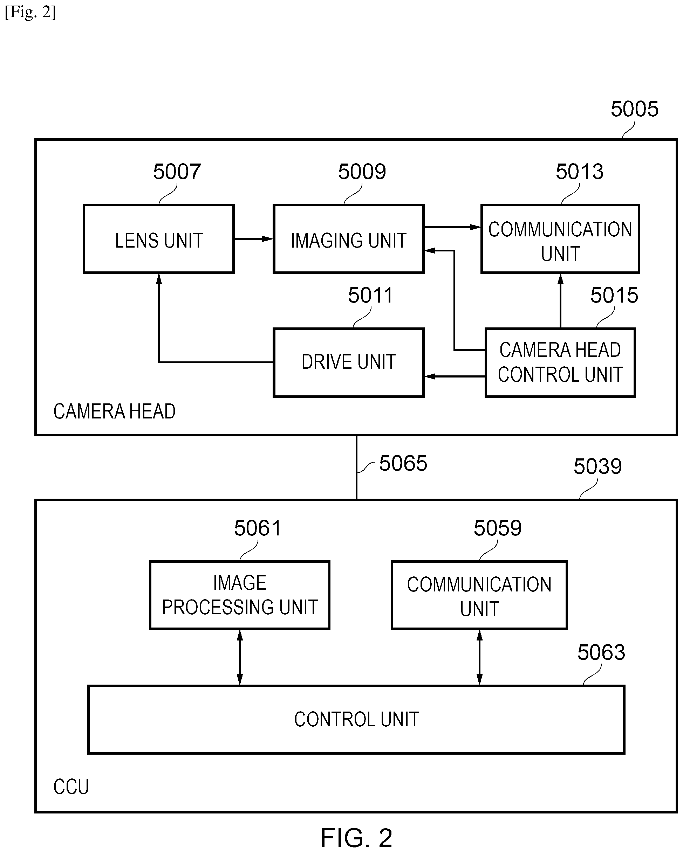

Functions of the camera head 5005 and the CCU 5039 of the endoscope 5001 will be described in more detail with reference to Fig. 2. Fig. 2 is a block diagram illustrating an example of functional configurations of the camera head 5005 and the CCU 5039 illustrated in Fig. 1.

The camera head 5005 has a lens unit 5007, an imaging unit 5009, a drive unit 5011, a communication unit 5013, and a camera head control unit 5015 as functions thereof with reference to Fig. 2.

Furthermore, the CCU 5039 has a communication unit 5059, an image processing unit 5061, and a control unit 5063 as functions thereof. The camera head 5005 and the CCU 5039 are connected to be capable of bi-directional communication via a transmission cable 5065.

First, the functional configuration of the camera head 5005 will be described. The lens unit 5007 is an optical system provided at a connection portion with the lens barrel 5003. Observation light taken in from the distal end of the lens barrel 5003 is guided to the camera head 5005 and is incident onto the lens unit 5007. The lens unit 5007 is configured by combining a plurality of lenses including a zoom lens and a focus lens. Optical characteristics of the lens unit 5007 are adjusted such that observation light is collected on a light receiving surface of an imaging element of the imaging unit 5009. Furthermore, the zoom lens and the focus lens are configured such that positions on the optical axis thereof can be moved for adjustment of magnification and a focal length of a captured image.

The imaging unit 5009 is constituted by the imaging element, and is arranged at the subsequent stage of the lens unit 5007. The observation light having passed through the lens unit 5007 is collected on the light receiving surface of the imaging element, and an image signal corresponding to the observation image is generated by photoelectric conversion. The image signal generated by the imaging unit 5009 is provided to the communication unit 5013.

As the imaging element constituting the imaging unit 5009, for example, a complementary metal oxide semiconductor (CMOS) type image sensor that is capable of color capturing having the Bayer arrangement can be used. Note that, for example, an imaging element capable of being compatible with capturing of a high-resolution image of 4K or more may be used as the imaging element. Since the high-resolution image of an operation site can be obtained, the operator 5067 can grasp a situation of the operation site in more detail and can proceed surgery more smoothly.

Furthermore, the imaging element constituting the imaging unit 5009 is configured to have a pair of imaging elements to acquire image signals for a right eye and a left eye, respectively, compatible with 3D display. As the 3D display is performed, the operator 5067 can more accurately grasp a depth of a living tissue in the operation site. Note that a plurality of the lens units 5007 is provided to correspond to the respective imaging elements in a case where the imaging unit 5009 is configured in a multi-plate type.

Furthermore, the imaging unit 5009 is not necessarily provided in the camera head 5005. For example, the imaging unit 5009 may be provided inside the lens barrel 5003 just behind an objective lens.

The drive unit 5011 is configured using an actuator, and the zoom lens and the focus lens of the lens unit 5007 are moved along the optical axis by a predetermined distance under the control of the camera head control unit 5015. With the movement, the magnification and the focal length of the image captured by the imaging unit 5009 can be appropriately adjusted.

The communication unit 5013 is configured using a communication device to transmit and receive various types of information to and from the CCU 5039. The communication unit 5013 transmits an image signal obtained from the imaging unit 5009 as RAW data to the CCU 5039 via the transmission cable 5065. In this case, it is preferable that the image signal be transmitted by optical communication in order to display the captured image of the operation site with low latency. During surgery, the operator 5067 performs the surgery while observing a state of the affected site through the captured image, and thus, it is required to display a moving image of the operation site in real time as much as possible in order for a safer and more reliable surgery. In the case where the optical communication is performed, a photoelectric conversion module that converts an electric signal into an optical signal is provided in the communication unit 5013. The image signal is converted into the optical signal by the photoelectric conversion module, and then, is transmitted to the CCU 5039 via the transmission cable 5065.

Furthermore, the communication unit 5013 receives a control signal to control the drive of the camera head 5005 from the CCU 5039. The control signal includes information regarding imaging conditions such as information to designate a frame rate of a captured image, information to designate an exposure value at the time of imaging, and/or information to designate magnification and a focal length of a captured image, for example. The communication unit 5013 provides the received control signal to the camera head control unit 5015. Note that a control signal from the CCU 5039 may also be transmitted by optical communication. In this case, the communication unit 5013 is provided with a photoelectric conversion module that converts an optical signal into an electric signal, and the control signal is converted into the electrical signal by the photoelectric conversion module, and then, is provided to the camera head control unit 5015.

Note that the imaging conditions such as the above-described frame rate, exposure value, magnification, and focal length are automatically set by the control unit 5063 of the CCU 5039 on the basis of the acquired image signal. That is, the endoscope 5001 is equipped with so-called auto exposure (AE) function, auto focus (AF) function, and auto white balance (AWB) function.

The camera head control unit 5015 controls the drive of the camera head 5005 on the basis of the control signal from the CCU 5039 received via the communication unit 5013. For example, the camera head control unit 5015 controls the drive of the imaging element of the imaging unit 5009 on the basis of the information to designate the frame rate of the captured image and/or the information to designate the exposure at the time of imaging. Furthermore, for example, the camera head control unit 5015 appropriately moves the zoom lens and the focus lens of the lens unit 5007 via the drive unit 5011 on the basis of the information to designate the magnification and the focal length of the captured image. Moreover, the camera head control unit 5015 may have a function of storing information to identify the lens barrel 5003 and the camera head 5005.

Note that the camera head 5005 can be made resistant to autoclave sterilization processing by arranging the configurations of the lens unit 5007, the imaging unit 5009, and the like in a sealed structure with high airtightness and waterproofness.

Next, the functional configuration of the CCU 5039 will be described. The communication unit 5059 is configured using a communication device to transmit and receive various types of information to and from the camera head 5005. The communication unit 5059 receives an image signal transmitted from the camera head 5005 via the transmission cable 5065. In this case, the image signal can be suitably transmitted by optical communication as described above. In this case, the communication unit 5059 is provided with a photoelectric conversion module that converts an optical signal into an electric signal to be compatible with the optical communication. The communication unit 5059 provides the image signal that has been converted into the electric signal to the image processing unit 5061.

Furthermore, the communication unit 5059 transmits a control signal to control the drive of the camera head 5005 to the camera head 5005. The control signal may also be transmitted by optical communication.

The image processing unit 5061 performs various types of image processing on the image signal which is RAW data transmitted from the camera head 5005. For examples, the image processing includes various types of known signal processing such as development processing, image quality improvement processing (band enhancement processing, super-resolution processing, noise reduction (NR) processing and/or camera shake correction processing, for example), and/or enlargement processing (electronic zoom processing). Furthermore, the image processing unit 5061 performs the detection processing on an image signal for performing AE, AF, and AWB.

The image processing unit 5061 is configured using a processor such as a CPU and a GPU, and the above-described image processing and detection processing can be performed when the processor operates according to a predetermined program. Note that, in a case where the image processing unit 5061 is constituted by a plurality of GPUs, the image processing unit 5061 appropriately divides information regarding the image signal and performs the image processing in parallel by the plurality of GPUs.

The control unit 5063 performs various types of control regarding imaging of an operation site using the endoscope 5001 and display of such a captured image. For example, the control unit 5063 generates a control signal to control the drive of the camera head 5005. At this time, in a case where an imaging condition is input by a user, the control unit 5063 generates the control signal on the basis of the input by the user. Alternatively, in a case where the endoscope 5001 is equipped with the AE function, the AF function, and the AWB function, the control unit 5063 appropriately calculates optimal exposure value, focal length, and white balance to generate the control signal in accordance with a result of the detection processing by the image processing unit 5061.

Furthermore, the control unit 5063 causes the display device 5041 to display the image of the operation site on the basis of the image signal subjected to the image processing by the image processing unit 5061. At this time, the control unit 5063 recognizes various objects in the image of the operation site using various image recognition technologies. For example, the control unit 5063 detects a shape of an edge, a color, and the like of an object included in the operation site image, and thus, can recognize a surgical tool such as forceps, a specific living body part, bleeding, mist at the time of using the energy treatment tool 5021, and the like. When the display device 5041 is caused to display the image of the operation site, the control unit 5063 causes various types of surgical support information to be superimposed and displayed on the image of the operation site using such a recognition result. Since the surgical support information is superimposed and displayed, and presented to the operator 5067, it is possible to proceed the surgery more safely and reliably.

The transmission cable 5065 connecting the camera head 5005 and the CCU 5039 is an electric signal cable compatible with communication of an electric signal, an optical fiber compatible with optical communication, or a composite cable thereof.

Here, communication is performed in a wired manner using the transmission cable 5065 in the illustrated example, but the communication between the camera head 5005 and the CCU 5039 may be performed in a wireless manner. In the case where the communication between the two is performed in a wireless manner, it is not necessary to lay the transmission cable 5065 in the operating room, and thus, a situation in which movement of a medical staff is hindered by the transmission cable 5065 in the operating room can be resolved.

An example of the endoscopic surgery system 5000 to which the technology according to the present disclosure can be applied has been described as above. Note that the endoscopic surgery system 5000 has been described as an example here, but a system to which the technology according to the present disclosure can be applied is not limited to such an example. For example, the technology according to the present disclosure may be applied to a flexible endoscope system for inspection or a microscopic surgery system.

Alternatively, aspects of the present disclosure may be applied to a medical robot system including a master-slave medical robot system. In the medical robot system, a user (such as doctor 5067) operates a master apparatus (surgeon console) to transmit an operation command to a slave apparatus (bedside cart) through a wired or wireless communication means and remotely operate the slave apparatus. The medical robot system may also includes a separate cart that contains some supporting hardware and software components, such as an electrosurgical unit (ESU), suction/irrigation pumps, and light source for the endoscope/microscope.

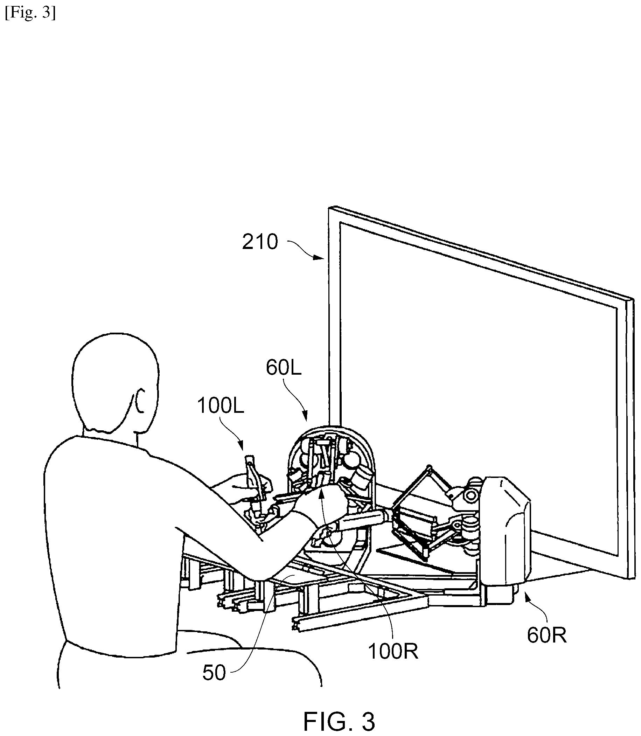

Fig. 3 illustrates a use example of the master apparatus 60 according to the present disclosure. In Fig. 3, two master apparatuses 60R and 60L for a right hand and a left hand are both provided. A surgeon puts both arms or both elbows on the supporting base 50, and uses the right hand and the left hand to grasp the operation portions 100R and 100L, respectively. In this state, the surgeon operates the operation portions 100R and 100L while watching a monitor 210 showing a surgical site. The surgeon may displace the positions or directions of the respective operation portions 100R and 100L to remotely operate the positions or directions of surgical instruments attached to slave apparatuses each of which is not illustrated, or use each surgical instrument to perform a grasping operation.

The basic configuration of example surgery systems applicable to embodiments of the disclosure has been described above with reference to Figs. 1 to 4 of the present disclosure. Hereinafter, specific embodiments of the present disclosure will be described.

### <Apparatus for controlling an image capture device during surgery>

As noted above, it is desirable that an apparatus is provided which reduces the delay in the provision of critical visual information to a surgeon or robotic surgeon during surgery due to dynamic changes in the surgical environment. Accordingly, an apparatus for controlling an image capture device during surgery is provided in accordance with embodiments of the disclosure.

The apparatus for controlling an image capture device during surgery may be applied to an example endoscopic surgical procedure such as that described with reference to Fig. 1 of the present disclosure. Accordingly, embodiments of the present disclosure will be described with reference to this example surgical procedure. However, it will be appreciated that the present disclosure is not intended to be limited to this specific surgical procedure. Rather, embodiments of the disclosure may be applied to any such surgical procedure. In fact, embodiments of the disclosure are applicable to any surgical procedure involving computer assisted surgical systems and devices.

### <Example Situation>

Returning now to Fig. 1 of the present disclosure. In this example surgical procedure, the doctor (or herein, surgeon) 5067 is performing endoscopic surgery on patient 5071. The surgeon 5067 cannot see inside the body cavity of the patient 5071 with their own eyes. Rather, the surgeon relies on the image captured by the endoscopic device 5001 which is displayed on the display screen 5041. As such, in this example, the endoscopic device captures images of the surgical scene and provides those images to the surgeon. This enables the surgeon to perform surgical operations inside the body cavity of the patient 5071 using surgical tools (such as the energy treatment tool 5021) even though the surgeon 5067 cannot see directly inside the body cavity of the patient.

In this sense, the endoscopic device captures critical visual information of the surgical scene for display to the surgeon.

Now, in the example situation of Fig. 1, the endoscopic device 5001 is supported by the support arm 5027. The support arm therefore holds the endoscopic device 5001 in position such that the endoscopic device captures an image of the surgical scene from an initial viewpoint. This initial viewpoint may be determined by the surgeon 5067 prior to the surgeon 5067 commencing surgery. Movement of the support arm 5027 to position the endoscopic device 5001 at the initial location (corresponding to the initial viewpoint) to provide this first viewpoint of the surgical scene is controlled by the arm control device 5045.

Consider that, during surgery, the surgeon 5067 inserts the energy treatment tool 5021into the body cavity of the patient. This may be necessary in order to perform a certain step of the surgical procedure. The surgeon 5067 is guided during the insertion of the energy treatment tool by the image of the surgical scene captured by the endoscopic device 5001.

In this example situation, once the energy treatment tool 5021 has been inserted to the desired location within the patient, the surgeon 5067 then begins to operate the energy treatment tool 5021.

During operation, the energy treatment tool 5021 heats up a target area of the patient. This may be done by the surgeon 5067 in order to cauterise a bleed. It will be appreciated that use of the energy treatment tool may produce a mist or smoke within the body cavity of the patient 5071. This mist or smoke may obscure the view of the surgical scene from the viewpoint of the endoscopic device 5001 such that the endoscopic device can no longer obtain a clear image of the surgical scene. As such, while using the energy treatment tool 5021, the surgeon (viewing the image captured by the endoscopic device 5001 on the display screen 5041) may no longer be able to see a clear image of the surgical scene inside the patient.

In other words, because, during operation of the energy treatment tool 5021, the endoscopic device 5001 is no longer able to capture a clear image of the surgical scene from its initial viewpoint, the surgeon may be not able to receive critical visual information from inside the body cavity of the patient. As such, during this time, there is a risk that the surgeon will not identify the occurrence of an important surgical event (such as a further surgical bleed) while the energy treatment tool 5021 is being operated.

As such, the surgeon may have to stop operation of the energy treatment tool 5021 until the mist and/or smoke clears such that a clear image of the scene is restored on the display device 5041. However, this delays the progress of the surgical procedure. Alternatively, the surgeon 5067 (or, alternatively, a human or computer assistant of the surgeon 5067) may have to control the endoscopic device 5001 in order to attempt to reconfigure the endoscopic device 5001 such that a clear image of the scene can be obtained despite the mist and/smoke which has been produced by the operation of the energy treatment tool 5021. However, while this enables the surgeon 5067 to continue with the surgical procedure, there will be a delay between the loss of the clear image of the scene (owing to the mist and/or smoke) and the restoration of a clear image of the scene. During this interval, important safety critical visual information regarding the surgical scene may be missed by the surgeon.

As such, according to embodiments of the disclosure, an apparatus for controlling an image capture device during surgery is provided in accordance with embodiments of the disclosure.

### Apparatus:

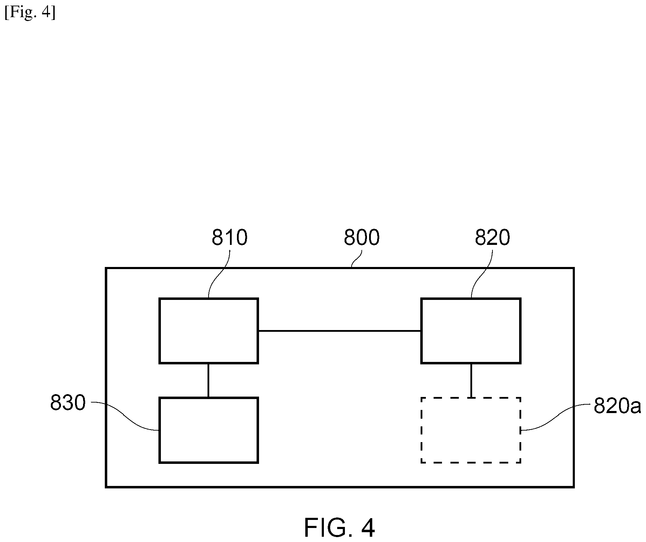

Fig. 4 illustrates an apparatus/system for controlling an image capture device, such as a medical image capture device, during surgery in accordance with embodiments of the disclosure.

The apparatus 800 includes an acquiring unit 810 configured to acquire first image data from the medical image capture device, the first image data being of an appearance of a surgical scene at a first instance of time; a determining unit 820, based on a predicted appearance of the surgical scene based on the first image data at a second instance of time after the first instance of time, one or more desired image capture properties of the medical image capture device; and a controlling unit 830, configured to control the image capture device at a third instance of time, the third instance of time being between the first instance of time and the second instance of time, in accordance with the one or more desired image capture properties of the medical image capture device.

In certain example embodiments, controlling the image capture device includes controlling the position of an articulated arm supporting the image capture device. That is, an articulated arm supporting the image capture device may be controller by the apparatus/system 800 in order to control the position of the image capture device in the surgical scene. That is, returning to the example situation of Fig. 1 of the present disclosure, the apparatus 800 may be connected to the arm control device 5045 in order to control the movement of the endoscopic device 5001. Alternatively, the apparatus 800 may be connected to, or form part of, the CCU 5039.

In certain examples, the apparatus 800 may, as illustrated in Fig. 4, optionally include a generating unit 820a configured to generate, in accordance with the first image data, second image data, the second image data being of the predicted appearance of the surgical scene at the second instance of time after the first instance of time. This enables the second image data, being of the predicted appearance of the surgical scene at the second instance of time, to be stored and used in subsequent processes (such as training of the machine learning system).

Operations of the apparatus 800 will now be described with reference to the example endoscopic surgical situation as illustrated with reference to Fig. 1 of the present disclosure. However, it will be appreciated that the apparatus may be applied to any such surgical situation as required.

### Acquiring Unit:

During surgery, the acquiring unit 810 of apparatus 800 acquires a first image (or image data) from the endoscopic device 5001 of the surgical scene. This first image provides the apparatus 800 with information regarding the appearance of the surgical scene at the time the image was captured by the endoscopic device 5001. In this example, this first image is the same image that is displayed to the surgeon on the display device 5041. That is, the first image shows the current appearance of the surgical scene.

It will be appreciated that the manner by which the acquiring unit 810 acquires the first image data is not particularly limited. For example, the acquiring unit 810 can acquire the image data from an image capture device (such as the endoscopic device 5001) by any suitable wired or wireless means. Moreover, the actual form of the image data will depend upon the type of image capture device which is used to capture the image data. In the present example (described with reference to Fig. 1 of the present disclosure) the image capture device is endoscopic device 5001. As such, in this example, the image data acquired by the acquiring unit 810 may be a high definition image, 4K image or 8K image of the scene.

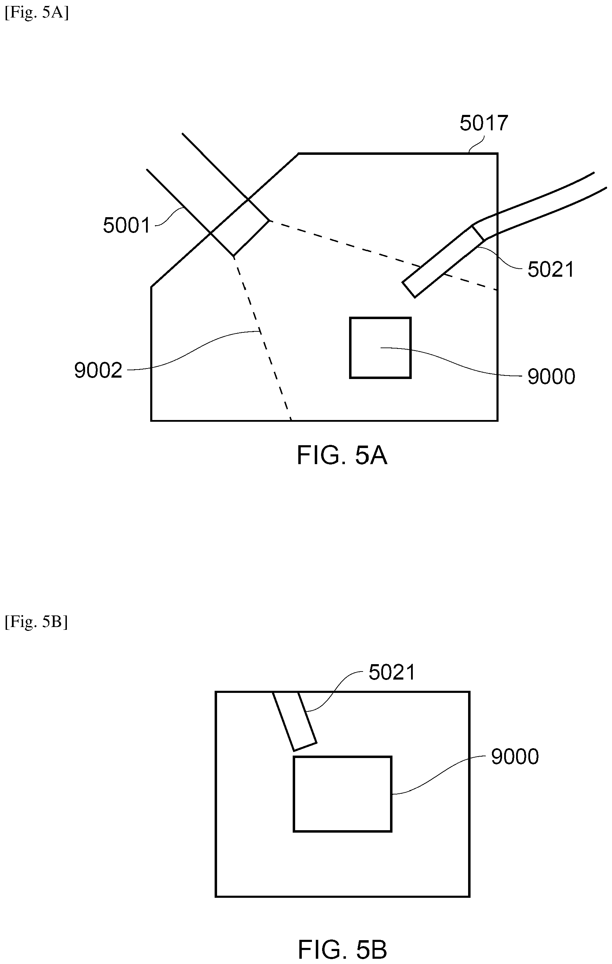

Now consider that, in this example situation, the surgical procedure has progressed to a stage whereby the surgeon 5067 has just inserted the energy treatment tool 5021 into the body cavity of the patient. This example surgical scene is illustrated in Fig. 5A of the present disclosure.

In Fig. 5A, a target treatment region 9000 within the body cavity of patient 5017 is shown. Energy treatment tool 5021 (which has been inserted by the surgeon 5067) is shown approaching the target treatment region 9000. Endoscopic device 5001 captures an image of the surgical scene from its first viewpoint. The region of the surgical scene which is captured by the endoscopic device 5001 is illustrated by region 9002. The image of this region captured by the endoscopic device 5001 is acquired by apparatus 800 and forms the first image of the surgical scene.

At this stage, the first image acquired by apparatus 800 includes a clear image of the surgical scene; that is, the first image will provide an unobstructed view of the surgical scene (specifically, of the target treatment region 9000) from the viewpoint of the endoscopic device 5001. An example illustration of this first image data is shown in Fig. 5B. As can be seen from the illustration in Fig. 5B, the image from the viewpoint of the endoscopic device 5001 will include the target treatment region 9000 and at least a portion of the energy treatment tool 5021.

### Determining Unit:

According to embodiments of the disclosure, determining unit 820 of apparatus 800 is then configured to determine, based on the predicted appearance of the surgical scene based on the first image data at a second instance of time after the first instance of time, one or more desired image capture properties of the image capture device 5001.

The time step or time gap between the time of the first image and the time for which the predicted appearance of the first image of the scene is determined will vary in accordance with the situation. The duration of the time gap may be set in advance during the initial configuration of the apparatus 800. Alternatively, the time gap may be dynamically adjusted by the apparatus 800 or the surgeon 5067 during the surgical procedure.

However, in this example, the time gap may be approximately 1 second. Time gaps much shorter and much larger than this example time gap are envisaged.

In other words, in this example, apparatus 800 determines the predicted appearance of the surgical scene approximately 1 second in the future. This predicted appearance is determined in accordance with the first image data which has been acquired (showing the current appearance of the surgical scene).

### <Predicted Appearance>

In certain examples, the determining unit 820 may receive a predicted image of the scene from an external computational device or server. In other embodiments, however, the determining unit 820 of the apparatus 800 generates a predication of how the visual characteristics of the surgical scene will appear after a time step (i.e. the predicted data or image data) using a scene prediction algorithm.

The actual form of the predicted data (being the predicted appearance of the surgical scene) is not particularly limited, and will vary in accordance with the situation to which the embodiments of the disclosure are applied. In certain examples, however, as discussed in more detail below, the predicted data will consist of a similar data type to the image data acquired by the acquiring unit 810 (that is, image sensor pixel values such as RGB pixel values in the case that the image acquired by the acquiring unit 810 consists also of such). Moreover, while the certain examples are described with the predicted data including a single predicted appearance, it will be understood that multiple predictions may alternatively be made, such that the predicated appearance data includes the predicted appearance of the surgical scene at sequentially increasing time steps.

Advantageously, when the predicted appearance of the scene is in a similar form to the acquired image data (namely, when the predicted appearance of the scene forms a predicted image of the scene) advanced image processing techniques may be used by apparatus 800 in order to analyse the content of the predicted image. Analysis of the predicted image by the determining unit in order to determine the one or more desired image capture properties of the image capture device is described in more detail below.

Now, in embodiments, the prediction algorithm used by the determining unit 820 to generate the predicted appearance of the scene may include a known machine learning algorithm such as a generative adversarial network (GAN). However, it will be appreciated that the present disclosure is not particularly limited to GAN machine learning algorithms, and any such machine learning algorithm may be used as required. These machine learning algorithms can generate a realistic prediction of the appearance of a scene at a future time based on a database of past video and image data of similar scenarios. Any new image which is provided to the algorithm then generates a prediction of the appearance of the scene in the near future (that is, at a given time step in the future from the image provided to the algorithm).

In the example of a surgical environment, the database of past video and image data used to train the machine learning algorithm may include videos and images of previous surgeries performed by the surgeon (being either a computer assisted surgical system or a human surgeon). The training database may also include videos and image of previous surgeries performed by other surgeons. In some situations, the training database may also include validated photorealistic simulations of a surgical scene. These validated photorealistic simulations of a surgical scene may be produced specifically for the purpose of training the machine learning algorithm. The training database may also include depth data showing the depth information of the surgical scene. The training database may also include configuration data of the articulated arm supporting the image capture device and/or data relating to a posture of the articulated arm such as rotation angle of a joint of the arm, a length of a link jointed by the joint of the arm, for example. That is, the machine learning system can be trained on any surgical data obtained in surgical scenarios as required.

In certain examples, deep learning models may be used in order to generate the realistic predicted appearance data. These deep learning models are constructed using neural networks. These neural networks include an input layer and an output layer. A number of hidden layers are located between the input layer and the output layer. Each layer includes a number of individual nodes. The nodes of the input layer are connected to the nodes of the first hidden layer. The nodes of the first hidden layer (and each subsequent hidden layer) are connected to the nodes of the following hidden layer. The nodes of the final hidden layer are connected to the nodes of the output layer. In other words, each of the nodes within a layer connect back to all the nodes in the previous layer of the neural network.

Of course, it will be appreciated that both the number of hidden layers used in the model and the number of individual nodes within each layer may be varied in accordance with the size of the training data and the individual requirements of the predicted data.

Now, each of the nodes takes a number of inputs, and produces an output. The inputs provided to the node (through connections with the previous layers of the neural network) have weighting factors applied to them.

In a neural network, the input layer receives a number of inputs (which can include the surgical data obtained in surgical scenarios as noted above). That is, in this example, the input layer may receive one or more of: images of past surgical scenarios, validated simulations of surgical scenarios and/or prior images of the present surgical scenario information regarding actions taken by a surgeon during previous surgical scenarios and/or image capture properties of a medical image capture device used during previous surgical scenarios as the input to the input layer. These inputs are then processed in the hidden layers, using weights that are adjusted during the training. The output layer then produces a prediction from the neural network.

Specifically, during training, the training data may be split into inputs and targets. The input data is all the data except from the target (being the appearance of the image of the surgical scene which the model is being trained to predict). The input data is then analysed by the neural network during training in order to adjust the weights between the respective nodes of the neural network. In examples, the adjustment of the weights during training may be achieved through linear regression models. However, in other examples, non-linear methods may be implemented in order to adjust the weighting between nodes to train the neural network.

Effectively, during training, the weighting factors applied to the nodes of the neural network are adjusted in order to determine the value of the weighting factors which, for the input data provided, produces the best match to the target data. That is, during training, both the inputs and target outputs are provided. The network then processes the inputs and compares the resulting output against the target data. Differences between the output and the target data are then propagated back through the neural network, causing the neural network to adjust the weights of the respective nodes of the neural network (backpropagation).

Of course, the number of training cycles (or epochs) which are used in order to train the model may vary in accordance with the situation. In some examples, the model may be continuously trained on the training data until the model produces an output within a predetermined threshold of the target data.

Once trained, new input data can then be provided to the input layer of the neural network, which will cause the model to generate (on the basis of the weights applied to each of the nodes of the neural network during training) a predicted output for the given input data (such as the predicted appearance of the surgical scene at a certain time step in the future).

Of course, it will be appreciated that the present embodiment is not particularly limited to the deep learning models (such as the neural network) and any such machine learning algorithm can be used in accordance with embodiments of the disclosure depending on the situation.

Moreover, the actual data which is used in order to train the machine learning algorithm is not particularly limited and will vary in accordance with the type of surgical scenario to which the embodiments of the disclosure will be applied.

It is possible to automatically train predictive networks on a large pool of videos or images using an event recognition network (of course the other data obtained during a surgery may also be used). For example, the predictive network may output a prediction for a given image or sequence of images for what comes next (that is, for how the next image in the sequence is predicted to appear). This prediction (e.g. the predicted data forming the predicted appearance of the scene) would then be declared correct or incorrect by the event recognition network analysis for the subsequent images in the sequence. This enables the machine learning system to be automatically trained on a large database in a short amount of time, thus improving the quality of the predictions made by the machine learning system.

Now, based on the occurrence of events in the training database, and the current appearance of the surgical scene, the determining unit 820 can form a prediction of the appearance of the surgical scene at a future time. That is, from the training data, the determining unit 820 may, for example, learn that when a first surgical tool (such as a scalpel) is introduced into a scene, a second surgical tool (such as a suction device) is highly likely to be introduced into the scene a short time after the time at which the first surgical tool was introduced, in a similar location to the first surgical tool. Using this information, and the current appearance of the surgical scene, the determining unit 820 can then predict the appearance of the surgical scene a short time after the introduction of the scalpel.

Moreover, in embodiments, the determining unit 820 may be configured to determine and output confidence values of the prediction, which may be resolved for different parts of the predicted appearance of the surgical scene. Parts of the prediction with confidence values below a predetermined threshold value could then be excluded from the subsequent analysis. This ensures that only areas of the surgical scene which have been predicted with high levels of confidence (or certainty) are included in the subsequent analysis by apparatus 800, thus improving the accuracy of the apparatus 800.

Of course, while prediction algorithm used by the determining unit 820 may include a generative adversarial network, the present disclosure is not intended to be particularly limited in this respect. Alternatively, variational auto-encoders may be used to produce the predicted image in accordance with embodiments of the disclosure. Further alternatively, autoregressive models may be used as the prediction algorithm in accordance with embodiments of the disclosure.

In fact, any such method may be used by the determining unit 820 to produce the predicted appearance of the surgical scene in accordance with the acquired first image data as required depending on the situation.

As described above, in certain example embodiments, apparatus 800 may further include an optional generating unit 820a, which is configured to generate, in accordance with the first image data, second image data, the second image data being of the predicted appearance of the surgical scene (at the second instance of time).

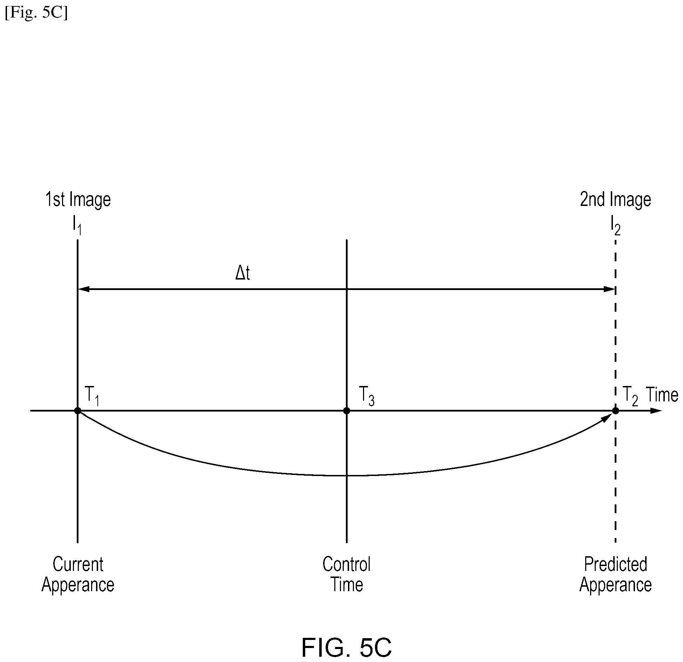

In this regard, Fig. 5C shows a time chart of the generation of the predicted appearance of a surgical scene at a future time. In Fig. 5C, time is shown on a horizontal axis and increases from left to right.

The first image data I1 (showing the current appearance of the surgical scene from the viewpoint of the endoscopic device 5001) is acquired by the apparatus 800 at time T1. At this time (T1) the apparatus 800 generates a second image data I2 which shows the predicted future appearance of the surgical scene at a time T2. The future time T2 for which the prediction of the appearance of the scene is made is a time Δt after the current time T1. As noted above, in this example, the time difference Δt may be approximately 1 second such that the second image data I2 shows the predicted appearance of the surgical scene at a time of approximately 1 second in the future.

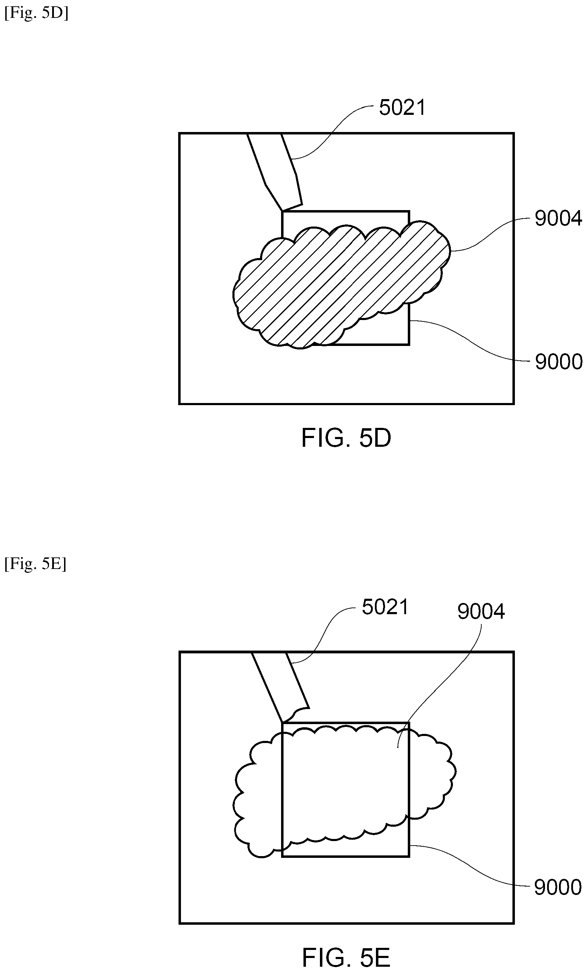

An example of the second image data I2 generated by the apparatus 800 is shown in Fig. 5D. In this example second image data I2, the predicted appearance of the surgical scene at a future time T2 is shown. In this predicted image generated by apparatus 800, it can be seen that it is predicted that a fog 9004 obscures the surgical site 9000 from the viewpoint of the endoscopic device 5001 at time T2 (that is, at a time Δt in the future from the current time T1).

In other words, in this example, apparatus 800 uses to first image data to identify the insertion of the energy treatment tool 5021 and, on the basis of this first image data, predicts that, at a time period Δt in the future, the surgical scene from the viewpoint of the endoscopic device 5001 will have the appearance of the predicted second image data I2 shown in Fig. 5D. That is, because of the presence of the energy treatment tool 5021, apparatus 800 can predict that a fog (such as mist and/or smoke) will obscure the view of the surgical site 9000 at a time of approximately 1 second in the future.

The predicted image data I2 may be used directly to determine the one or more desired image capture properties of the image. Alternatively, the generating unit 820a may first store the predicted image data in a memory, whereby it can be later retrieved as required.

Now, in the specific surgical scene illustrated in of Fig. 5 of the present disclosure, apparatus 800 analyses the second image data I2 and determines that, because it is predicted that at time T2 the fog 9004 obscures the surgical site 9000, the surgeon 5067 may be prevented, by the fog, from observing critical visual information of the surgical site at the future time T2. Accordingly, apparatus 800 determines a configuration of image capture properties of the endoscopic device 5001 which are required in order that the loss of critical visual information owing to the predicted fog 9004 can be avoided.

That is, in this example, apparatus 800 determines that it is desired that the endoscopic device 5001 changes from observing in the visual region of the electromagnetic spectrum to observing in the infrared region of the electromagnetic spectrum. This is because apparatus 800 determines that while the fog obscures the visual image of the surgical site, a clear image of the surgical site can still be obtained by observing the surgical site using a different portion of the electromagnetic spectrum (such as the infrared region). As such, a desired image capture property of the endoscopic device at time T2 is that the endoscopic device 5001 switches to capturing images in the infrared region of the electromagnetic spectrum prior to the occurrence of the second instance of time.

### <Image Capture Properties>

The one or more desired image capture properties of the image capture device may include the determination of a desired imaging condition of the image capture device. The imaging condition of the image capture device may include one or more of an optical image system condition and an image processing condition. For example, the optical image system condition and/or the image processing condition may include one or more of a desired image zoom, an image focus, an image aperture, an image contrast and/or an image brightness. That is, an optical image system condition may include an optical image zoom or the like. In contrast, an image processing condition may include a digital image zoom or the like, performed by image processing circuitry at time of image capture, or applied to a captured image in post-processing. Alternatively, or in addition, the one or more desired image capture properties of the image capture device may also include a desired location or movement of the image capture device. Of course, the present disclosure is not particularly limited in this respect, and any such desired image capture properties may be determined by the determining unit depending on the situation. Further alternatively, as described above, the image capture property may include an image capture type (such as whether to capture a visual image of the scene or a hyperspectral image of the scene (using information from across the electromagnetic spectrum)).

Now, in embodiments of the disclosure, the determining unit 820 of the apparatus 800 may use one or more camera property algorithms in order to determine the optimum one or more image capture properties of the image capture device (such as the endoscopic device 5001) for the predicted appearance of the surgical scene.

The one or more image capture property algorithms used in order to determine the one or more image capture properties may, in examples, consist of a machine learning system which has been trained on input data including past surgical videos, validated simulations, data measured during a surgery by a surgeon (such as position information of the surgical tools and cameras, an environment map (which may be generated by simultaneous localization and mapping (SLAM)) and tool type information. That is, a machine learning algorithm trained on an appropriate training set may be used in order to determine the desired image capture properties of the image capture device.

Furthermore, a deep learning algorithm, including a neural network (such as the neural network described in detail above) can be used in accordance with embodiments of the disclosure in order to determine the one or more image capture properties of the image capture device.

In addition, data which has been labelled (either manually or automatically) may also be used to train the machine learning algorithm. The label data may include an evaluation result of steering, navigation or control of an imaging device during a surgical workflow. The label data may also include an evaluation of the visibility of important features such as active tools and events within the surgical scene (such as a bleed). This labelling may be performed by a surgeon 5067 or other medical staffs. Moreover, label data may include other goals relating to the usability of the image by a human visual system, such as viewpoint stability, consistent orientation and lighting. In this manner, the machine learning algorithm can learn to determine desired image capture properties which are optimised for use by the surgeon 5067.

Optionally, the algorithm to determine desired image capture properties may be rules based, where the predicted appearance of the surgical scene (e.g. the predicted image data) may be analysed in order to detect the occurrence of known scenarios, in combination with current image data and contextual data.

Desired image capture properties may then be determined using a lookup table of ideal camera properties and behaviours for the detected scenario. For a surgical scene, some example detected scenarios (or events) within the predicted image data and the resultant desired image capture properties are as follows:

### 1. Movement of a tool or object within the scene

The movement of a tool or object within the scene may cause view interference. That is, for example, movement of a tool (such as a suction tool) may obscure, or partially obscure, the image from the viewpoint of the image capture device in the scene. According to embodiments of the disclosure, given the position of the tool in the predicted image data, a desired imager movement may be calculated which avoids the obstruction but maintains the view of the surgical scene and tools.

### 2. Movement of a tool which is actively in-use

The future movement of a tool within the predicted image data may be used to define a desired imager movement which maintains the tool in the centre of the captured image, in an automatic camera follow function. The status of the tool (in-use or not in-use) may be detected based on data indicating the status and changed by an activation switch of the tool and so on.

### 3. Organ holding/slipping

Predicted image data may depict events where organs held by a retractor may be dropped or may move. A desired imager movement may be selected where the movement of the organ may be tracked within the image, keeping both the retractor and organ in the field of view. Alternatively, maintaining the organ in the field of view may be achieved by changing the level of zoom used by the image capture device.

### 4. Predicted bleeding event

A desired imager movement may be selected where the bleed origin location is as close as possible to the centre of the captured image while maintaining other critical image features within the field of view, such as the surgical tools. This may be achieved with a combination of lateral and angular change in camera position.

### 5. Tool change

A desired image capture property may be selected when a tool change occurs (that is, when a tool which has been used is removed, or when a new tool is introduced into the scene). The desired image capture properties will be determined such that the area into which the tool will be introduced is visible, while maintaining visibility of other critical features within the predicted image data. For example, a camera angle may be selected which gives visibility of the region below the trocar entrance into the patient's body cavity while maintaining a view of the surgical scene.

### 6. Change in a pulling direction of retraction (by non-dominant hand)

There would be a change in direction/position of exfoliation/ablation and the tool would move to a portion to which a tension is caused by a tool hold by non-dominant hand. The desired image capture properties of the image capture device could be determined in order to take account of this change.

It will be appreciated that there are many other surgical scenarios which could be used in a lookup table to determine the one or more desired image capture properties of the image capture device in accordance with embodiments of the disclosure, and that the present disclosure is not intended to be limited to these aforementioned examples.

While the present determining unit has been described using both machine learning algorithms and lookup tables, it will be appreciated that any suitable method may be used by the determining unit 820 in order to determine the desired image capture properties of the image capture device as required. The skilled person may, for example, apply existing techniques for automated camera position systems to the predicted image data generated by the generating unit 820a in order to determine desired image capture properties such as the desired movement of the image capture device or a desired image capture position.

It will be appreciated there may be a situation in which the predicted image data may include multiple scenarios or events. That is, the predicted image data may include a number of predictions of a high likelihood, but which actually require very different image capture properties. In certain examples, it may thus be desired that the camera or image capture device uses image capture properties which capture all of the possible outcomes, or which allow the camera to occupy the ideal viewpoint for each scenario quickly, as soon as its occurrence is confirmed. For example, this may be at the halfway point between two desired viewpoints for different scenarios.

Furthermore, in certain examples, the determining unit 820 may be configured to calculate a weighting for image capture properties of the image capture device in accordance with one or more factors associated with the image capture properties, and determine the image capture properties having the highest weighting factor as the desired image capture properties for the image capture device. That is, a range of image capture properties could be produced (such as a range of viewing positions) and each of those viewing positions weighted in accordance with factors such as an advantage (e.g. how well that position enables the surgeon to view the target region of the surgical scene, or what percentage of the image is taken up by the target region of the surgical scene). The image capture property (or, in this example, position) with the highest computed weighting would then be deemed the most advantageous image capture property and would be chosen as the desired image capture property for the surgical scene.

Alternatively, the weighting for the image capture properties of the image capture device is based on a comparison of the image capture properties of the image capture device with a set of target image capture properties of the image capture device. The closer the image capture properties of the image capture device are to the target image capture properties, the higher the weighting factor would be.

### Controlling Unit:

Once the determining unit has determined the desired image capture properties of the image capture device, the controlling unit is configured to control the image capture device at a third instance of time, the third instance of time being between the first instance of time and the second instance of time, in accordance with the one or more desired image capture properties of the image capture device.

As such, in this example, apparatus 800 controls the endoscopic device 5001 at a third instance of time, the third instance of time being between the first instance of time and the second instance of time, in accordance with the one or more desired image capture properties of the endoscopic device (namely, that the endoscopic device captures images using the infrared portion of the electromagnetic spectrum).

Returning to Fig. 5C, the third instance of time T3 (the control time) is shown on the time chart at a time between the current time T1 and the second time T2 (being the time at which it is predicted that the fog will obscure the image). As such, apparatus 800 will, at time T3, control the endoscopic device such that the endoscopic device switches to capturing images using the infrared portion of the electromagnetic spectrum. The actual location of the time T3 on the time chart is not particularly limited, provided that it is between the current time T1 and the time for which the prediction is made T2 and provided that it is sufficiently ahead of the time T2 such that the image capture properties of the endoscopic device can be adjusted by apparatus 800 to correlate with the desired image capture properties that have been determined before the second time T2 is reached.

As such, in this example, when the second time T2 is reached (and the surgeon 5067 has, as predicted, started using the energy treatment tool 5021) the apparatus 800 will have controlled the endoscopic device 5001 such that the endoscopic device 5001 captures images not in the visual region of the electromagnetic spectrum, but rather in the infrared region of the electromagnetic spectrum. As such, at time T2 the actual image which is captured by the endoscopic device 5001 shows a clear image of the surgical site 5001 despite the presence of the mist and/or smoke which is produced by the operation of the energy treatment tool 5021. An illustration of the actual image which is obtained by the endoscopic device 5001 at the time T2 (that is, after the control of the image capture properties of the endoscopic device 5001) is shown in Fig. 5E.

In embodiments, the controlling unit is configured to compare the current image capture properties of the image capture device (such as endoscope 5001) to the desired image capture properties of the image capture device and, subsequently, use the comparison of these properties to create image controller instructions which will cause the image capture device to achieve the desired image capture properties at the desired time. For example, a comparison of the current location of the image capture device to the desired location of the image capture device enables the controlling unit to determine corresponding actuation instructions which can be used to move the image capture device to the desired location.

### Advantageous Effects:

According to embodiments of the disclosure, the apparatus for controlling an image capture device during surgery enables a computer assisted surgical system to predictively optimise the image capture properties of a camera, such that the impact of predictable future changes to the surgical scene are accounted for through adjustment of the image capture properties and/or configuration of the image capture device, before the predicted change actually occurs in the surgical scene. In this manner, negative changes to the imaging of the surgical scene (such as a disruption of the provision of critical image information to the surgeon) can be avoided without delay to the surgical procedure.

Of course, the present disclosure is not particularly limited to these advantageous technical effects, there may be others as will become apparent to the skilled person when reading the present disclosure.

### Additional Modifications:

It will be appreciated that the image capture device may include any medical image capture device as required in accordance with the situation. That is, while configurations of the apparatus 800 have been described above with reference to Figs. 5 to 6 of the present disclosure, it will be appreciated that the embodiments of the disclosure are not limited to this specific example. For example, while the embodiments of the disclosure have been described with reference to an endoscopic imaging device, the embodiments of the disclosure may also be applied to a telescope imager, a microscope imager, an exoscopic imager or the like, as required. Furthermore, a number of additional modifications to the configuration of the apparatus are described below.

### Context Sensing System:

While the above described methods used by the determining unit to generate the predicted appearance of the scene utilize the acquired image data to generate the prediction, it will be appreciated that the present disclosure is not particularly limited in this regard. Rather, certain additional information may be used by apparatus 800 during the production of the predicted data and the determination of the one or more desired image capture properties of the image capture device. This additional information may, in certain examples, provide contextual information which further enhances the predictive ability of the apparatus 800.

In some examples, the contextual information may be provided to the generating unit in order to assist in the generation of the predicted images of the scene. For example, the scene prediction algorithm may rely on cues from the contextual information to generate depicted changes to the scene, where a plurality of data sources may be used to generate any particular example of the predicted image data. Alternatively, the contextual sensing system may be configured to perform analysis of the contextual information, with this analysis being used by the determining unit 820 when determining the desired image capture properties of the image capture device. Further alternatively, the contextual information may be provided directly to the determining unit such that the determining unit incorporates the contextual information into its own analysis.

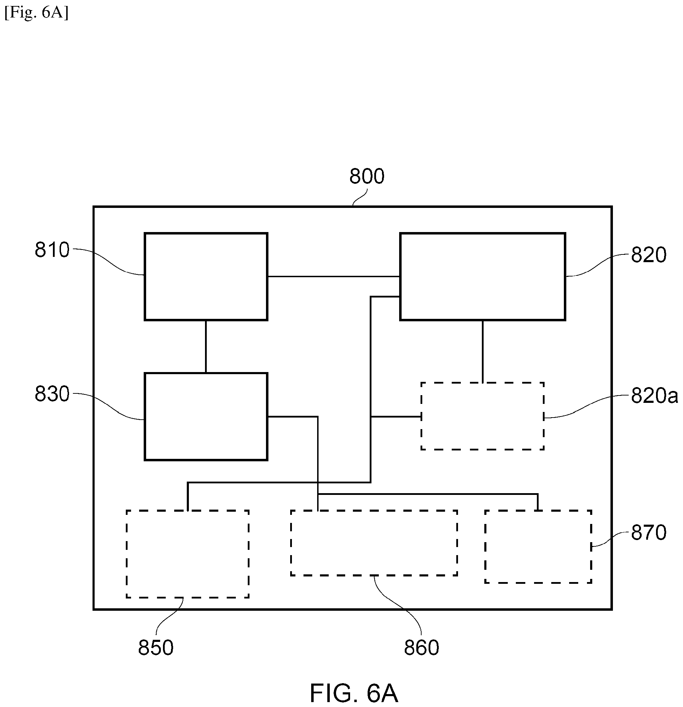

As such, optionally, according to embodiments of the disclosure, the acquiring unit 810 may include a context sensing system 850 configured to collect additional information regarding the surgical context which may be relevant to changes occurring in the surgical scene. This is illustrated in Fig. 6A of the present disclosure.

The context sensing system may include a number of context sensing means (including a range of distinct cameras and sensors) configured to collect contextual information regarding the surgical procedure which is being performed by the surgeon. The contextual information obtained by the context sensing means may include at least one of: the position of an object in the scene, the movement of an object in the scene, a type of object which is present in the scene and/or an action being performed by a person in the scene.

However the configuration of the context sensing system 850 is therefore not particularly limited and will vary in accordance with the specific situation to which the embodiments of the disclosure are applied.

A number of specific examples of contextual information which can be obtained by the context sensing means of the context sensing system 850 are provided below.

In examples, the context sensing system 850 may include a camera and/or microphone within the operating room but being outside of the patient's body. The camera and/or microphone will monitor the events which occur in the operating room outside the patient's body. Sound recordings from the microphone may enable the context sensing system to monitor conversations within the operating room (including instructions from the surgeon such as, "please pass me the forceps" or, "we have a bleed"). Alternatively, images from the camera of the context sensing system may enable the context sensing system to determine the orientation of a surgical bed, the room lighting, the relative positions of staff within the operating room and the like. Alternatively, data from the microphone and the camera could be used by the context sensing unit in order to determine the condition of staff within the operating room, such as stress level or current task level engagement of the operating staff.

Monitoring the environment outside the patient's body using a camera and/or microphone therefore allows the apparatus 800 to gain a greater contextual awareness of the progress of the surgical procedure, which may enhance both the predicative ability of the generating unit 820a and the selection of the image capture properties by the determining unit 820.

Alternatively, or in addition, the context sensing system may include patient sensors such as blood pressure, breathing and heart rate sensors and the like. These sensors can provide additional contextual information regarding the status of the patient who is being operated on. For example, a drop in blood pressure of the patient could indicate that a bleed has occurred; this information can be used by the generating unit 820a and the determining unit 820 when producing the predicted image and the desired image capture properties in order further improve the ability of the apparatus 800 to maintain a clear image of the surgical scene. Alternatively, contextual information regarding tissue movement related rhythms (such as the heart beat), breathing cycles, abdominal air pressure and the like could be monitored by discrete sensors of the context sensing system 850 in order to further enhance the determination of the image capture properties of the image capture device by apparatus 800.

Alternatively, or in addition, tools which communicate current tool activity condition or other parameters could be coupled with the context sensing system. For example, the context sensing system can be configured to receive status updates from surgical tools (such as the energy treatment tool 5021). These status updates, which could be received by a wired or wireless interface, can inform the context sensing system of whether the tool is active. In the example of the energy treatment tool, the context sensing system 850 can know that, when the energy treatment tool is active, there may be an increase in the amount of mist and/or smoke within the body cavity of the patient. The apparatus 800 can then determine the desired image capture properties of the image captured device accordingly.

Alternatively, the contextual system 850 could include one or more sensors and/or circuitry configured to determine which tool is being prepared by the assistant surgeon for use in a next stage of the surgical procedure; the number of tool changes that have occurred within a certain period of time; tool type information, (including information about how the tool is manipulated by the surgeon); tool motion information, such as the velocity and trajectory of the tool; information regarding how the tool is being held by the surgeon or the support staff; information regarding tool conditions and operation settings which may relate to imminent visual changes (the amount of suction/irrigation/aspiration, for example) and the like.

This additional contextual information can be used in order to assist the apparatus 800 in the determination of the image capture properties for the image capture device. For example, the context sensing unit can use the information received from the tools as a cue which is used in order to enhance the accuracy of the predicted image of the scene generated by the generating unit 820a.

Alternatively or in addition, the context sensing system 850 could be configured to receive manual input by the surgeon or other medical staff. Manual input could be received by the context sensing system 850 through operation of a touch screen device or computer keyboard, for example. This information could be used to inform the apparatus 800 of the current stage of the surgical procedure (such as, "entering stage two"). Determining the stage of the surgical procedure in this manner assists the apparatus 800 in the determination of image capture properties of the image capture device. For example, by knowing which stage of the surgical procedure has been started, the apparatus 800 can more accurately determine and predict which tools are likely to be introduced to, or removed from, the surgical scene.

### Virtual Camera Unit:

In examples, the apparatus 800 may further include a virtual viewpoint system 860. The virtual viewpoint system 860 can be used in order to create a virtual viewpoint which has a coordinate position in 3D space which may be separate from the camera position (that is, the virtual camera position). In other words, a synthesised virtual viewpoint is generated through images captured between movements of the image capture device, with the virtual viewpoint being distinct and offset from the actual location of the image capture device at a given instance of time. In order to produce the virtual viewpoint, the virtual viewpoint system 860 is configured to combine image data from the movements of the image capture device using image stitching and viewpoint virtualisation algorithms which are known in the art.

In this manner, the virtual viewpoint system 860 may act autonomously to control the real camera position of the image capture apparatus, while a human controller controls the virtual camera position. In examples, the virtual camera position may be fully autonomously controlled, where it can fulfil image stability goals of the human visual system, while rapidly collecting data using autonomous control of the image capture device.

In examples where a virtual camera viewpoint is being used, an enhanced virtual camera viewpoint may be predicted by apparatus 800 which will benefit the surgeon's view of the scene. Moreover, the real camera movement required to generate the virtual viewpoint, can be determined by the determining unit 820 as one or more desired image capture properties of the image capture device.

### Capability Sensing Unit:

In examples, the apparatus 800 may further include a capability sensing unit 870. The capability sensing unit 870 may be configured to interact with the determining unit 820 and controlling unit 830 in order to determine the optimum image capture properties which can be achieved for a given set of desired image capture properties. That is, the capability sensing unit 870 may determine that one or more restrictions or limitations of the image capture device or the surgical environment prevent the image capture device from achieving the desired image capture properties which have been determined by the determining unit 820 within the required time frame. In this case, the capability sensing unit 870 will instruct the controlling unit 830 of the optimum image capture properties which can be achieved by the image capture device. In other words, the capability sensing unit will account for a limitation of the image capture device when determining the desired/optimum image capture properties for the image capture device.

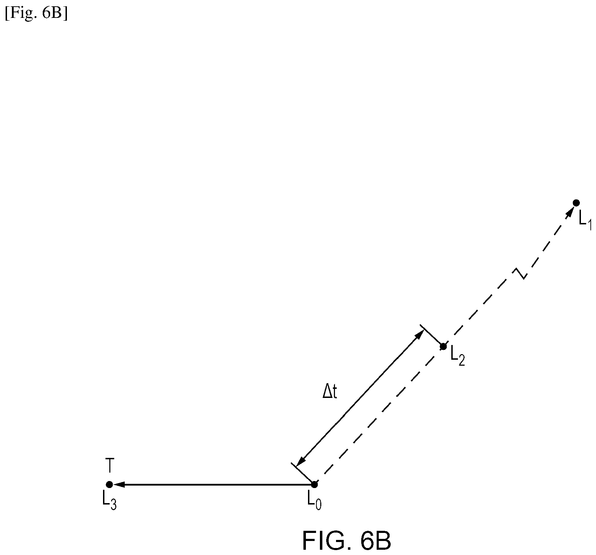

Consider the example whereby the desired image capture property of the image capture device is a desired image capture location. This is illustrated in Fig. 6B of the present disclosure. In this example, apparatus 800 has produced a desired location L1 for the image capture device to capture images from at a future time T. When the desired location is determined (by the determining unit 820) the capability sensing unit 870 will assess capabilities of the image capture device (including the current location of the image capture device, L0, and the maximum velocity V of the image capture device) in order to determine whether the image capture device can achieve the desired location L1 by time T (which is a time Δt in the future).

However, in this example, the capability sensing unit determines that the image capture device will not achieve the location L1 by time T. Rather, based on the capabilities of the image capture device, the image capture device will only achieve a location L2 by time T. As such, the capability sensing unit determines that the image capture device cannot achieve location L1 by time T.

However, location L2 is a suboptimal location for the time T compared to location L1. That is, in contrast to L1, location L2 may not provide a clear view of the scene. As such, the capability sensing unit interacts with the determining unit 820 in order to determine the optimal location for the image capture device within the movement range of the image capture device that can be achieved within the time step Δt. On this basis, it is determined that location L3 is the optimal location of the image capture device which can be achieved within the time step Δt (that is, which can be achieved by time T).

Accordingly, since, owing to the limitations of the capability of the image capture device, it is determined that the image capture device cannot achieve the desired location L1 a second desired location L3 within the capabilities of the image capture device is determined. This avoids the image capture device arriving at a suboptimal location L2 owing to its inability to achieve the desired location L1 within the requisite time.

Moreover, it will be appreciated that the capability sensing unit may also consider other restrictions, such as environmental restrictions, when determining the whether the image capture device can achieve the desired image capture properties. For example, the capability sensing unit may determine that a desired image capture location cannot be achieved by the image capture device owing to the potential for collision with a tissue of the patient or a surgical tool of the surgeon, or the like.

Optionally, the capability sensing unit 870 may be implemented in accordance with one or more rules of the surgical scenario. That is, the apparatus may recognise a particular surgical scenario (such as heart surgery) through the acquired image data and/or the acquired contextual data. The recognition of the surgical scenario may then be used to query a lookup table of pre-defined constraints which may be applied to possible movements or desired image capture properties of the image capture device. In heart surgery, for example, it may be a constraint that a certain portion of the heart is retained within the field of view of the image capture device, or that the position of the image capture device does not impede the surgeon's ability to access a certain portion of the heart.

In examples of embodiments of the disclosure, the capability sensing means 870 and/ or the controlling unit 830 may be configured in order to determine the movement pattern to the desired image capture location in accordance with the location of one or more objects present in the scene. This ensures that the image capture device does not collide with an object within the scene on the way to the desired location.

Specifically, in some embodiments, once the movement pattern to the desired image capture location has been determined, the controlling unit is configured to control the position and/or orientation of an articulated arm supporting the image capture device in accordance with the determined movement pattern in order to efficiently move the image capture device to the desired location without negatively impacting the surgical scene (through a collision with an object within the scene, for example) on route to the desired location.

Of course, while the capability of the image capture device has been illustrated with reference to the desired location, it will be appreciated that the capability sensing unit may also be applied to other desired image capture properties of the image capture device as required.

### Example Setup:

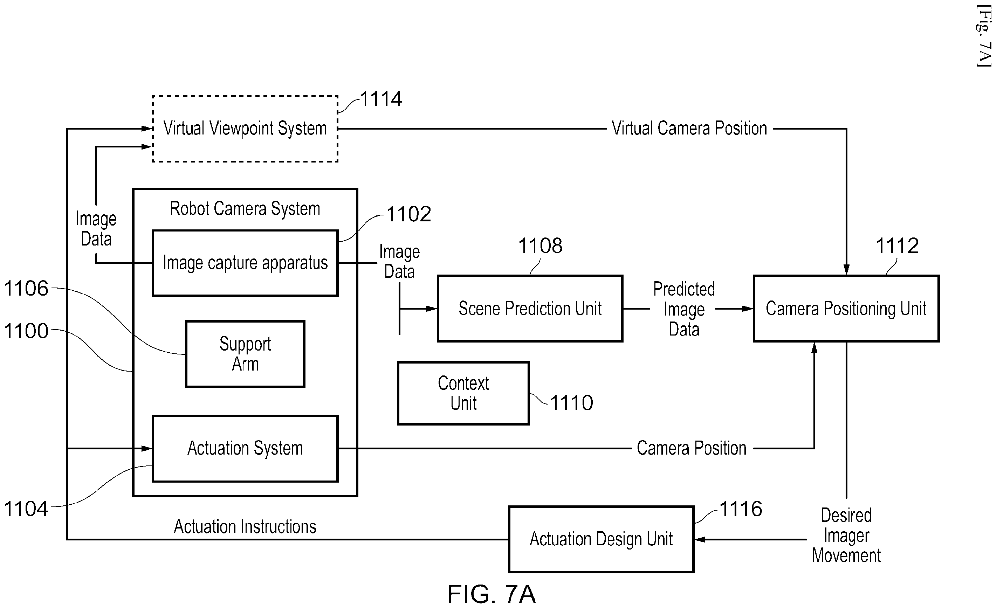

An example setup of a computer assisted surgical system in accordance with embodiments of the present disclosure is illustrated with reference to Fig. 7A of the present disclosure. This example set up may be used in an endoscopic surgical situation (as described with reference to Fig. 1 of the present disclosure), for example.

In this example setup, a robotic camera system 1100 is provided. This robotic camera system includes an image capture apparatus 1102 and an actuation system 1104. The image capture apparatus is coupled to the actuation system by a support arm 1106. In other words, the actuation system 1104 is used to move the support arm 1106 in order to position the image capture device 1102 such that the image capture device captures an image of the surgical scene.

An image acquiring unit (not shown) acquires image data from the image capture device and provides this image data to a scene prediction unit (such as generating unit 820a of apparatus 800). Furthermore, a context sensing unit 1100 acquires contextual information of the surgical scene and provides this contextual information to the scene prediction unit 1108.

The scene prediction unit 1108 then, on the basis of this information, generates a predicted image data indicative of the appearance of the surgical scene at a time T in the future (that is, at a time T ahead of the time at which the image data was captured by the image capture apparatus of the robot camera system).

This predicted image data is provided to a camera positioning unit 1112 (such as determining unit 820 of apparatus 800). The camera positioning unit 1112 also receives a virtual camera position from the virtual viewpoint system 1114 (generated based on image data received from the image capture apparatus 1100).

Based on this information (and also, the current position of the support arm of the robot camera system 1100) the camera positioning unit 1112 computes a desired imager movement. This is provided to an actuation design unit 1116 (such as controlling unit 830 of apparatus 800) which designs a series of actuation commands for provision to the actuation unit of the robot camera system to move the robot camera system to the desired location ahead to the time T.

In this manner, the position of the robot camera system can be controlled such that the robot camera system achieves a desired location ahead of a time T. This enables the computer assisted surgical system to predictively optimise the position of the camera, such that the impact of predictable future changes to the surgical scene is accounted for through movements of the camera systems before the changes occur.

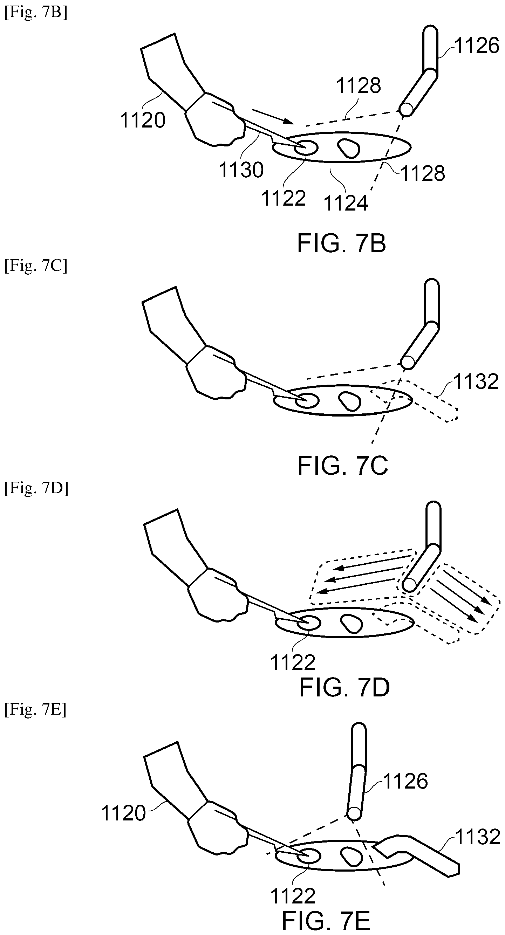

An example illustration of the application of this computer assisted surgical system to a surgical scenario is illustrated with reference to Fig. 7B. In Fig. 7B, a surgeon 1120 is performing a computer assisted surgical operation on a target region 1122 of a patient 1124. The surgeon 1120 watches an image of the surgical scene which has been captured by a robotic camera system 1126 on a display (not shown). The field of view of the robotic camera system is illustrated by the region encompassed by the lines 1128 in Fig. 7B. The images from the robotic camera system 1126 are also provided to the scene prediction unit 1108 as illustrated in Fig. 7A.

Consider, in this example, that the surgeon 1120 has just introduced a scalpel 1130 into the surgical scene. At this stage, the scene prediction unit (which may be the generating unit of apparatus 800) predicts that in the subsequent video frames, within a time of 2 seconds, the surgeon 1120 will introduce a suction tube 1132 into the surgical scene. This is illustrated in Fig. 7C. Analysis of the predicted image reveals that the predicted placement of the suction tube 1132 in the surgical scene will block the robotic camera system 1126, thus preventing a clear image of the scene from being obtained.

Accordingly, given the time step of the predicted image (namely, two seconds in advance of the current time) the camera positioning unit 1112 of Fig. 7A calculates the possible movements within a three dimensional volume surrounding the robotic camera system 1126 which can be achieved by the robotic camera system 1126 and which provide a clear image of the target region 1122. This is illustrated in Fig. 7D.

Finally, once the desired image capture location of the robotic camera system has been determined, the actuation design unit 1116 and actuation unit 1104 interact in order to control the robotic camera system 1126 such that the robotic camera system 1126 adjusts its location and angle in order to maintain a clear view of the target region 1122 before the surgeon 1120 actually places the suction tube 1132 into the surgical scene. This is illustrated in Fig. 7E.

### Method:

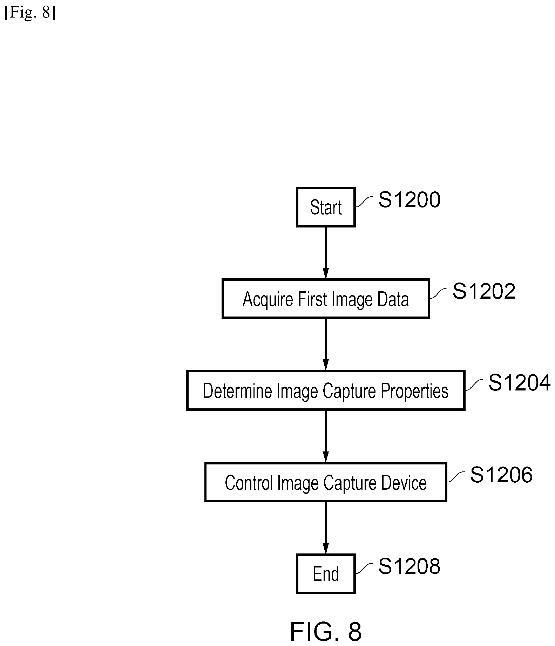

In accordance with embodiments of the disclosure, a method of controlling an image capture device during surgery is provided. An illustration of the method of controlling an image capture device during surgery in accordance with an embodiment of the disclosure is illustrated in Fig. 8.

The method of controlling an image capture device, such as a medical image capture device, during surgery starts at S1200 and proceeds to step S1202.

In step S1202, the method includes acquiring first image data from the medical image capture device, the first image data being of an appearance of a surgical scene at a first instance of time.

Once the first image data has been acquired, the method proceeds to step S1204.

In step S1204, the method includes determining, based on a predicted appearance of the surgical scene based on the first image data at a second instance of time after the first instance of time, one or more desired image capture properties of the medical image capture device.

Once the one or more desired image capture properties of the image capture device have been determined, the method proceeds to step S1206.

In step S1206, the method includes controlling the image capture device at a third instance of time, the third instance of time being between the first instance of time and the second instance of time, in accordance with the one or more desired image capture properties of the medical image capture device.

Once the image capture device has been controlled at the third instance of time, the method proceeds to, and ends with, step S1208.

It will be appreciated that, in some situations, the desired image capture properties of the image capture device may be those image capture properties that the image capture device already possesses. In this case, no change to the current image capture properties of the image capture device will be performed.

Moreover, it will be appreciated that in some situations, once step S1206 has been completed, the method will return to step S1202. In this manner, the desired image capture properties of the image capture device and be continuously or periodically assessed and updated as required.

### Computing Device:

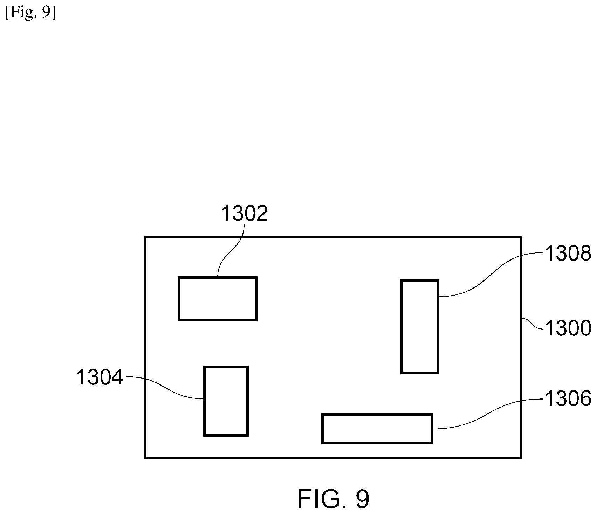

Referring now to Fig. 9, a computing device 1300 according to embodiments of the disclosure is shown. Computing device 1300 may be a computing device for controlling an image capture device during surgery. Typically, the computing device may be a device such as a personal computer or a terminal connected to a server. Indeed, in embodiments, the computing device may also be a server. The computing device 1300 is controlled using a microprocessor or other processing circuitry 1302.

The processing circuitry 1302 may be a microprocessor carrying out computer instructions or may be an Application Specific Integrated Circuit. The computer instructions are stored on storage medium 1304 which maybe a magnetically readable medium, optically readable medium or solid state type circuitry. The storage medium 1304 may be integrated into the computing device 1300 (as illustrated) or may be separate to the computing device 1300 and connected thereto using either a wired or wireless connection. The computer instructions may be embodied as computer software that contains computer readable code which, when loaded onto the processor circuitry 1302, configures the processor circuitry 1302 of the computing device 1300 to perform a method of controlling an image capture device during surgery according to embodiments of the disclosure.

Additionally connected to the processor circuitry 1302, is a user input (not shown). The user input maybe a touch screen or maybe a mouse or stylist type input device. The user input may also be a keyboard or any combination of these devices.

A network connection 1306 is also coupled to the processor circuitry 1302. The network connection 1306 may be a connection to a Local Area Network or a Wide Area Network such as the Internet or a Virtual Private Network or the like. The network connection 1306 may be connected to a medical device infrastructure allowing the processor circuitry 1302 to communicate with other medical devices in order to obtain relevant data or provide relevant data to the other medical devices. The network connection 1306 may be located behind a firewall or some other form of network security.

Additionally coupled to the processing circuitry 1302, is a display device 1308. The display device, although shown integrated into the computing device 1300, may additionally be separate to the computing device 1300 and may be a monitor or some kind of device allowing the user to visualise the operation of the system. In addition, the display device 1300 may be a printer or some other device allowing relevant information generated by the computing device 1300 to be viewed by the user or by a third party (such as medical support assistants).

Although the foregoing has been described with reference to a "master-slave" robotic system, the disclosure is not so limited. In some instances, the surgical robot may work independently of the human surgeon with the human surgeon being present in a supervisory capacity. Moreover, with endoscopy or laparoscopy, the scopist may be a robot with a human surgeon directing the robot. In embodiments, the robotic system may be a multi-robots surgical system where a main surgeon will use a robotic surgeon and an assistant surgeon will teleoperate assistive robotic arms. The robotic system may be a solo-surgery system which consists of a pair of co-operating and autonomous robotic arms holding the surgical instruments. In this case, the human surgeon may use a master-slave arrangement.

### <Example Systems>

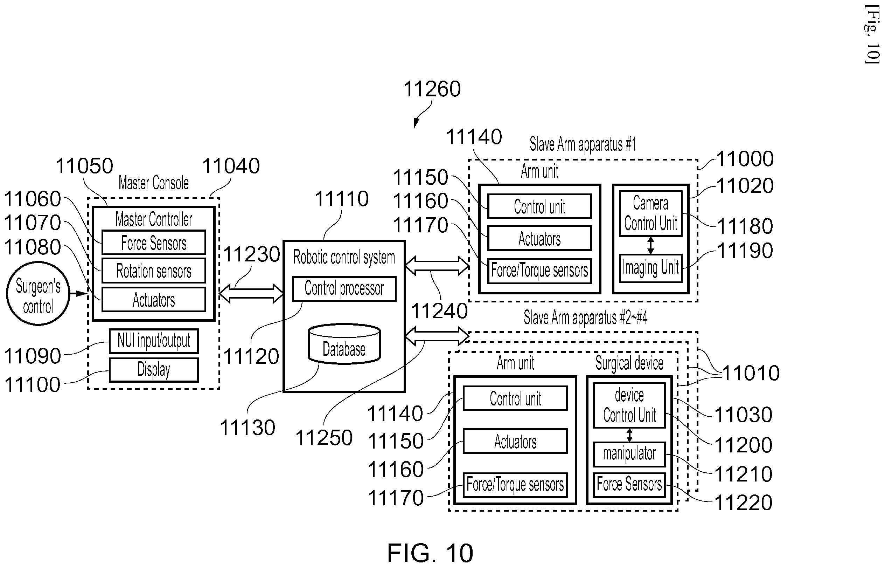

Fig. 10 schematically shows an example of a computer assisted surgery system 11260 to which the present technique is applicable. The computer assisted surgery system is a master slave system incorporating an autonomous arm 11000 and one or more surgeon-controlled arms 11010. The autonomous arm holds an imaging device 11020 (e.g. a medical scope such as an endoscope, microscope or exoscope). The one or more surgeon-controlled arms 11010 each hold a surgical device 11030 (e.g. a cutting tool or the like). The imaging device of the autonomous arm outputs an image of the surgical scene to an electronic display 11100 viewable by the surgeon. The autonomous arm autonomously adjusts the view of the imaging device whilst the surgeon performs the surgery using the one or more surgeon-controlled arms to provide the surgeon with an appropriate view of the surgical scene in real time.

The surgeon controls the one or more surgeon-controlled arms 11010 using a master console 11040. The master console includes a master controller 11050. The master controller 11050 includes one or more force sensors 11060 (e.g. torque sensors), one or more rotation sensors 11070 (e.g. encoders) and one or more actuators 11080. The master console includes an arm (not shown) including one or more joints and an operation portion. The operation portion can be grasped by the surgeon and moved to cause movement of the arm about the one or more joints. The one or more force sensors 11060 detect a force provided by the surgeon on the operation portion of the arm about the one or more joints. The one or more rotation sensors detect a rotation angle of the one or more joints of the arm. The actuator 11080 drives the arm about the one or more joints to allow the arm to provide haptic feedback to the surgeon. The master console includes a natural user interface (NUI) input / output for receiving input information from and providing output information to the surgeon. The NUI input / output includes the arm (which the surgeon moves to provide input information and which provides haptic feedback to the surgeon as output information). The NUI input may also include a voice input, a line of sight input and/or a gesture input. The master console includes the electronic display 11100 for outputting images captured by the imaging device 11020.

The master console 11040 communicates with each of the autonomous arm 11000 and one or more surgeon-controlled arms 11010 via a robotic control system 11110. The robotic control system is connected to the master console 11040, autonomous arm 11000 and one or more surgeon-controlled arms 11010 by wired or wireless connections 11230, 11240 and 11250. The connections 11230, 11240 and 11250 allow the exchange of wired or wireless signals between the master console, autonomous arm and one or more surgeon-controlled arms.

The robotic control system includes a control processor 11120 and a database 11130. The control processor 11120 processes signals received from the one or more force sensors 11060 and one or more rotation sensors 11070 and outputs control signals in response to which one or more actuators 11160 drive the one or more surgeon controlled arms 11010. In this way, movement of the operation portion of the master console 11040 causes corresponding movement of the one or more surgeon controlled arms.

The control processor 11120 also outputs control signals in response to which one or more actuators 11160 drive the autonomous arm 11000. The control signals output to the autonomous arm are determined by the control processor 11120 in response to signals received from one or more of the master console 11040, one or more surgeon-controlled arms 11010, autonomous arm 11000 and any other signal sources (not shown). The received signals are signals which indicate an appropriate position of the autonomous arm for images with an appropriate view to be captured by the imaging device 11020. The database 11130 stores values of the received signals and corresponding positions of the autonomous arm.

For example, for a given combination of values of signals received from the one or more force sensors 11060 and rotation sensors 11070 of the master controller (which, in turn, indicate the corresponding movement of the one or more surgeon-controlled arms 11010), a corresponding position of the autonomous arm 11000 is set so that images captured by the imaging device 11020 are not occluded by the one or more surgeon-controlled arms 11010.

As another example, if signals output by one or more force sensors 11170 (e.g. torque sensors) of the autonomous arm indicate the autonomous arm is experiencing resistance (e.g. due to an obstacle in the autonomous arm's path), a corresponding position of the autonomous arm is set so that images are captured by the imaging device 11020 from an alternative view (e.g. one which allows the autonomous arm to move along an alternative path not involving the obstacle).

It will be appreciated there may be other types of received signals which indicate an appropriate position of the autonomous arm.

The control processor 11120 looks up the values of the received signals in the database 11130 and retrieves information indicating the corresponding position of the autonomous arm 11000. This information is then processed to generate further signals in response to which the actuators 11160 of the autonomous arm cause the autonomous arm to move to the indicated position.

Each of the autonomous arm 11000 and one or more surgeon-controlled arms 11010 includes an arm unit 11140. The arm unit includes an arm (not shown), a control unit 11150, one or more actuators 11160 and one or more force sensors 11170 (e.g. torque sensors). The arm includes one or more links and joints to allow movement of the arm. The control unit 11150 sends signals to and receives signals from the robotic control system 11110.

In response to signals received from the robotic control system, the control unit 11150 controls the one or more actuators 11160 to drive the arm about the one or more joints to move it to an appropriate position. For the one or more surgeon-controlled arms 11010, the received signals are generated by the robotic control system based on signals received from the master console 11040 (e.g. by the surgeon controlling the arm of the master console). For the autonomous arm 11000, the received signals are generated by the robotic control system looking up suitable autonomous arm position information in the database 11130.

In response to signals output by the one or more force sensors 11170 about the one or more joints, the control unit 11150 outputs signals to the robotic control system. For example, this allows the robotic control system to send signals indicative of resistance experienced by the one or more surgeon-controlled arms 11010 to the master console 11040 to provide corresponding haptic feedback to the surgeon (e.g. so that a resistance experienced by the one or more surgeon-controlled arms results in the actuators 11080 of the master console causing a corresponding resistance in the arm of the master console). As another example, this allows the robotic control system to look up suitable autonomous arm position information in the database 11130 (e.g. to find an alternative position of the autonomous arm if the one or more force sensors 11170 indicate an obstacle is in the path of the autonomous arm).

The imaging device 11020 of the autonomous arm 11000 includes a camera control unit 11180 and an imaging unit 11190. The camera control unit controls the imaging unit to capture images and controls various parameters of the captured image such as zoom level, exposure value, white balance and the like. The imaging unit captures images of the surgical scene. The imaging unit includes all components necessary for capturing images including one or more lenses and an image sensor (not shown). The view of the surgical scene from which images are captured depends on the position of the autonomous arm.

The surgical device 11030 of the one or more surgeon-controlled arms includes a device control unit 11200, manipulator 11210 (e.g. including one or more motors and/ or actuators) and one or more force sensors 11220 (e.g. torque sensors).

The device control unit 11200 controls the manipulator to perform a physical action (e.g. a cutting action when the surgical device 11030 is a cutting tool) in response to signals received from the robotic control system 11110. The signals are generated by the robotic control system in response to signals received from the master console 11040 which are generated by the surgeon inputting information to the NUI input / output 11090 to control the surgical device. For example, the NUI input / output includes one or more buttons or levers comprised as part of the operation portion of the arm of the master console which are operable by the surgeon to cause the surgical device to perform a predetermined action (e.g. turning an electric blade on or off when the surgical device is a cutting tool).

The device control unit 11200 also receives signals from the one or more force sensors 11220. In response to the received signals, the device control unit provides corresponding signals to the robotic control system 11110 which, in turn, provides corresponding signals to the master console 11040. The master console provides haptic feedback to the surgeon via the NUI input / output 11090. The surgeon therefore receives haptic feedback from the surgical device 11030 as well as from the one or more surgeon-controlled arms 11010. For example, when the surgical device is a cutting tool, the haptic feedback involves the button or lever which operates the cutting tool to give greater resistance to operation when the signals from the one or more force sensors 11220 indicate a greater force on the cutting tool (as occurs when cutting through a harder material, e.g. bone) and to give lesser resistance to operation when the signals from the one or more force sensors 11220 indicate a lesser force on the cutting tool (as occurs when cutting through a softer material, e.g. muscle). The NUI input / output 11090 includes one or more suitable motors, actuators or the like to provide the haptic feedback in response to signals received from the robot control system 11110.

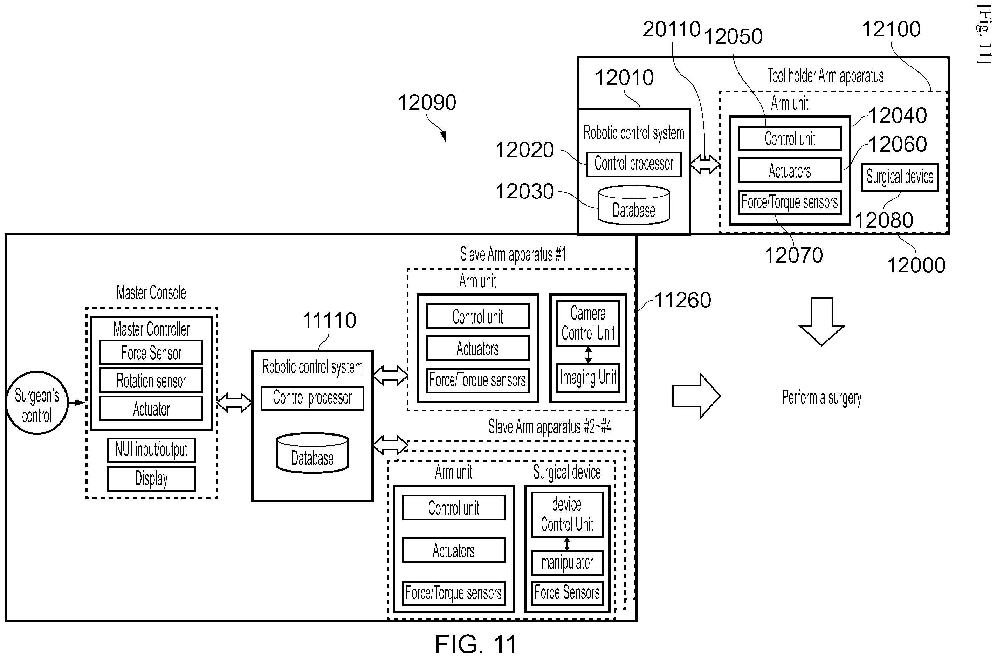

Fig. 11 schematically shows another example of a computer assisted surgery system 12090 to which the present technique is applicable. The computer assisted surgery system 12090 is a surgery system in which the surgeon performs tasks via the master slave system 11260 and a computerised surgical apparatus 12000 performs tasks autonomously.

The master slave system 11260 is the same as Fig. 10 and is therefore not described. The system may, however, be a different system to that of Fig. 10 in alternative embodiments or may be omitted altogether (in which case the system 12090 works autonomously whilst the surgeon performs conventional surgery).

The computerised surgical apparatus 12000 includes a robotic control system 12010 and a tool holder arm apparatus 12100. The tool holder arm apparatus 12100 includes an arm unit 12040 and a surgical device 12080. The arm unit includes an arm (not shown), a control unit 12050, one or more actuators 12060 and one or more force sensors 12070 (e.g. torque sensors). The arm includes one or more joints to allow movement of the arm. The tool holder arm apparatus 12100 sends signals to and receives signals from the robotic control system 12010 via a wired or wireless connection 12110. The robotic control system 12010 includes a control processor 12020 and a database 12030. Although shown as a separate robotic control system, the robotic control system 12010 and the robotic control system 11110 may be one and the same. The surgical device 12080 has the same components as the surgical device 11030. These are not shown in Fig. 11.

In response to control signals received from the robotic control system 12010, the control unit 12050 controls the one or more actuators 12060 to drive the arm about the one or more joints to move it to an appropriate position. The operation of the surgical device 12080 is also controlled by control signals received from the robotic control system 12010. The control signals are generated by the control processor 12020 in response to signals received from one or more of the arm unit 12040, surgical device 12080 and any other signal sources (not shown). The other signal sources may include an imaging device (e.g. imaging device 11020 of the master slave system 11260) which captures images of the surgical scene. The values of the signals received by the control processor 12020 are compared to signal values stored in the database 12030 along with corresponding arm position and/or surgical device operation state information. The control processor 12020 retrieves from the database 12030 arm position and/or surgical device operation state information associated with the values of the received signals. The control processor 12020 then generates the control signals to be transmitted to the control unit 12050 and surgical device 12080 using the retrieved arm position and/or surgical device operation state information.

For example, if signals received from an imaging device which captures images of the surgical scene indicate a predetermined surgical scenario (e.g. via neural network image classification process or the like), the predetermined surgical scenario is looked up in the database 12030 and arm position information and/or surgical device operation state information associated with the predetermined surgical scenario is retrieved from the database. As another example, if signals indicate a value of resistance measured by the one or more force sensors 12070 about the one or more joints of the arm unit 12040, the value of resistance is looked up in the database 12030 and arm position information and/or surgical device operation state information associated with the value of resistance is retrieved from the database (e.g. to allow the position of the arm to be changed to an alternative position if an increased resistance corresponds to an obstacle in the arm's path). In either case, the control processor 12020 then sends signals to the control unit 12050 to control the one or more actuators 12060 to change the position of the arm to that indicated by the retrieved arm position information and/or signals to the surgical device 12080 to control the surgical device 12080 to enter an operation state indicated by the retrieved operation state information (e.g. turning an electric blade to an "on" state or "off" state if the surgical device 12080 is a cutting tool).

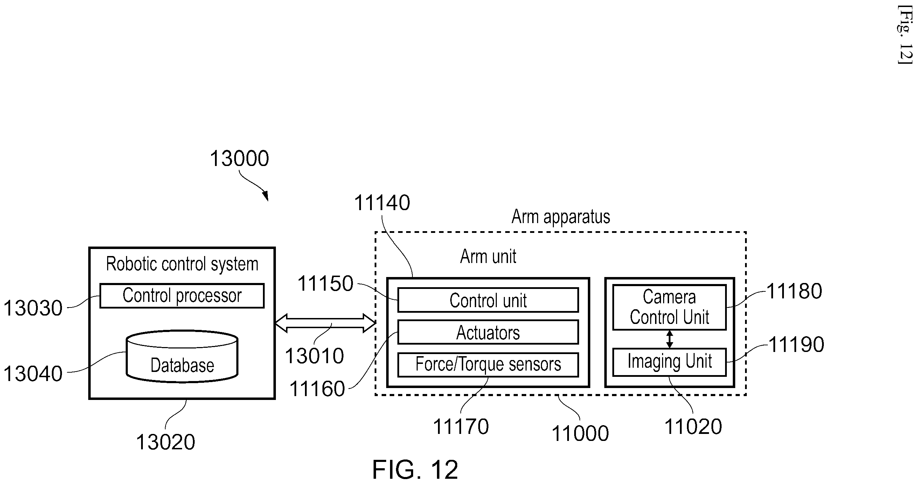

Fig. 12 schematically shows another example of a computer assisted surgery system 13000 to which the present technique is applicable. The computer assisted surgery system 13000 is a computer assisted medical scope system in which an autonomous arm 11000 holds an imaging device 11020 (e.g. a medical scope such as an endoscope, microscope or exoscope). The imaging device of the autonomous arm outputs an image of the surgical scene to an electronic display (not shown) viewable by the surgeon. The autonomous arm autonomously adjusts the view of the imaging device whilst the surgeon performs the surgery to provide the surgeon with an appropriate view of the surgical scene in real time. The autonomous arm 11000 is the same as that of Fig. 10 and is therefore not described. However, in this case, the autonomous arm is provided as part of the standalone computer assisted medical scope system 13000 rather than as part of the master slave system 11260 of Fig. 10. The autonomous arm 11000 can therefore be used in many different surgical setups including, for example, laparoscopic surgery (in which the medical scope is an endoscope) and open surgery.

The computer assisted medical scope system 13000 also includes a robotic control system 13020 for controlling the autonomous arm 11000. The robotic control system 13020 includes a control processor 13030 and a database 13040. Wired or wireless signals are exchanged between the robotic control system 13020 and autonomous arm 11000 via connection 13010.

In response to control signals received from the robotic control system 13020, the control unit 11150 controls the one or more actuators 11160 to drive the autonomous arm 11000 to move it to an appropriate position for images with an appropriate view to be captured by the imaging device 11020. The control signals are generated by the control processor 13030 in response to signals received from one or more of the arm unit 11140, imaging device 11020 and any other signal sources (not shown). The values of the signals received by the control processor 13030 are compared to signal values stored in the database 13040 along with corresponding arm position information. The control processor 13030 retrieves from the database 13040 arm position information associated with the values of the received signals. The control processor 13030 then generates the control signals to be transmitted to the control unit 11150 using the retrieved arm position information.

For example, if signals received from the imaging device 11020 indicate a predetermined surgical scenario (e.g. via neural network image classification process or the like), the predetermined surgical scenario is looked up in the database 13040 and arm position information associated with the predetermined surgical scenario is retrieved from the database. As another example, if signals indicate a value of resistance measured by the one or more force sensors 11170 of the arm unit 11140, the value of resistance is looked up in the database 12030 and arm position information associated with the value of resistance is retrieved from the database (e.g. to allow the position of the arm to be changed to an alternative position if an increased resistance corresponds to an obstacle in the arm's path). In either case, the control processor 13030 then sends signals to the control unit 11150 to control the one or more actuators 1116 to change the position of the arm to that indicated by the retrieved arm position information.

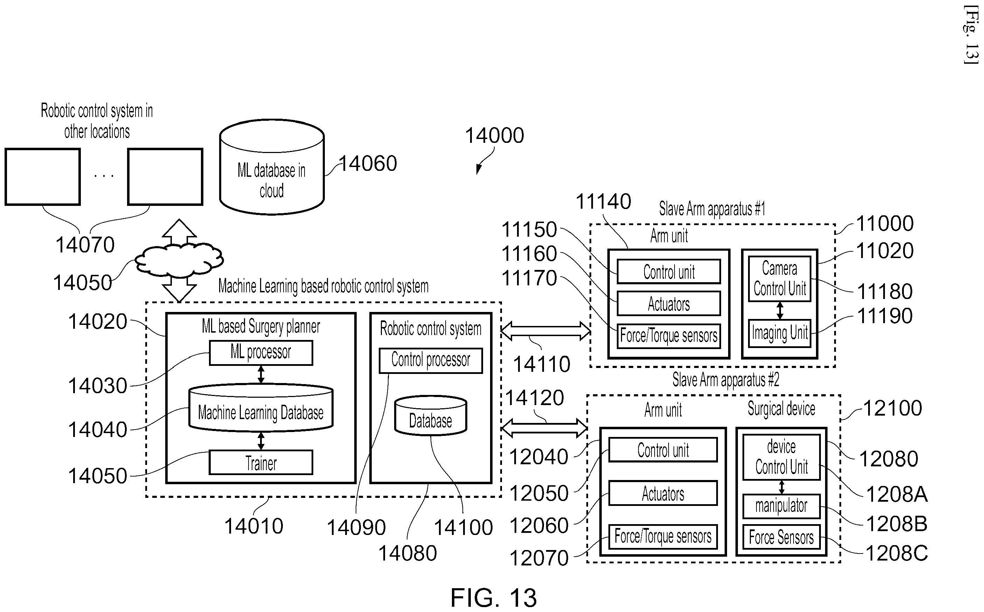

Fig. 13 schematically shows another example of a computer assisted surgery system 14000 to which the present technique is applicable. The system includes one or more autonomous arms 11000 with an imaging unit 11020 and one or more autonomous arms 12100 with a surgical device 12100. The one or more autonomous arms 11000 and one or more autonomous arms 12100 are the same as those previously described. Each of the autonomous arms 11000 and 12100 is controlled by a robotic control system 14080 including a control processor 14090 and database 14100. Wired or wireless signals are transmitted between the robotic control system 14080 and each of the autonomous arms 11000 and 12100 via connections 14110 and 14120, respectively. The robotic control system 14080 performs the functions of the previously described robotic control systems 11110 and/or 13020 for controlling each of the autonomous arms 11000 and performs the functions of the previously described robotic control system 12010 for controlling each of the autonomous arms 12100.

The autonomous arms 11000 and 12100 perform at least a part of the surgery completely autonomously (e.g. when the system 14000 is an open surgery system). The robotic control system 14080 controls the autonomous arms 11000 and 12100 to perform predetermined actions during the surgery based on input information indicative of the current stage of the surgery and/or events happening in the surgery. For example, the input information includes images captured by the image capture device 11000. The input information may also include sounds captured by a microphone (not shown), detection of in-use surgical instruments based on motion sensors comprised with the surgical instruments (not shown) and/or any other suitable input information.

The input information is analysed using a suitable machine learning (ML) algorithm (e.g. a suitable artificial neural network) implemented by machine learning based surgery planning apparatus 14020. The planning apparatus 14020 includes a machine learning processor 14030, a machine learning database 14040 and a trainer 14050.

The machine learning database 14040 includes information indicating classifications of surgical stages (e.g. making an incision, removing an organ or applying stitches) and/or surgical events (e.g. a bleed or a patient parameter falling outside a predetermined range) and input information known in advance to correspond to those classifications (e.g. one or more images captured by the imaging device 11020 during each classified surgical stage and/or surgical event). The machine learning database 14040 is populated during a training phase by providing information indicating each classification and corresponding input information to the trainer 14050. The trainer 14050 then uses this information to train the machine learning algorithm (e.g. by using the information to determine suitable artificial neural network parameters). The machine learning algorithm is implemented by the machine learning processor 14030.

Once trained, previously unseen input information (e.g. newly captured images of a surgical scene) can be classified by the machine learning algorithm to determine a surgical stage and/or surgical event associated with that input information. The machine learning database also includes action information indicating the actions to be undertaken by each of the autonomous arms 11000 and 12100 in response to each surgical stage and/or surgical event stored in the machine learning database (e.g. controlling the autonomous arm 12100 to make the incision at the relevant location for the surgical stage "making an incision" and controlling the autonomous arm 12100 to perform an appropriate cauterisation for the surgical event "bleed"). The machine learning based surgery planner 14020 is therefore able to determine the relevant action to be taken by the autonomous arms 11000 and/or 12100 in response to the surgical stage and/or surgical event classification output by the machine learning algorithm. Information indicating the relevant action is provided to the robotic control system 14080 which, in turn, provides signals to the autonomous arms 11000 and/or 12100 to cause the relevant action to be performed.

The planning apparatus 14020 may be included within a control unit 14010 with the robotic control system 14080, thereby allowing direct electronic communication between the planning apparatus 14020 and robotic control system 14080. Alternatively or in addition, the robotic control system 14080 may receive signals from other devices 14070 over a communications network 14050 (e.g. the internet). This allows the autonomous arms 11000 and 12100 to be remotely controlled based on processing carried out by these other devices 14070. In an example, the devices 14070 are cloud servers with sufficient processing power to quickly implement complex machine learning algorithms, thereby arriving at more reliable surgical stage and/or surgical event classifications. Different machine learning algorithms may be implemented by different respective devices 14070 using the same training data stored in an external (e.g. cloud based) machine learning database 14060 accessible by each of the devices. Each device 14070 therefore does not need its own machine learning database (like machine learning database 14040 of planning apparatus 14020) and the training data can be updated and made available to all devices 14070 centrally. Each of the devices 14070 still includes a trainer (like trainer 14050) and machine learning processor (like machine learning processor 14030) to implement its respective machine learning algorithm.

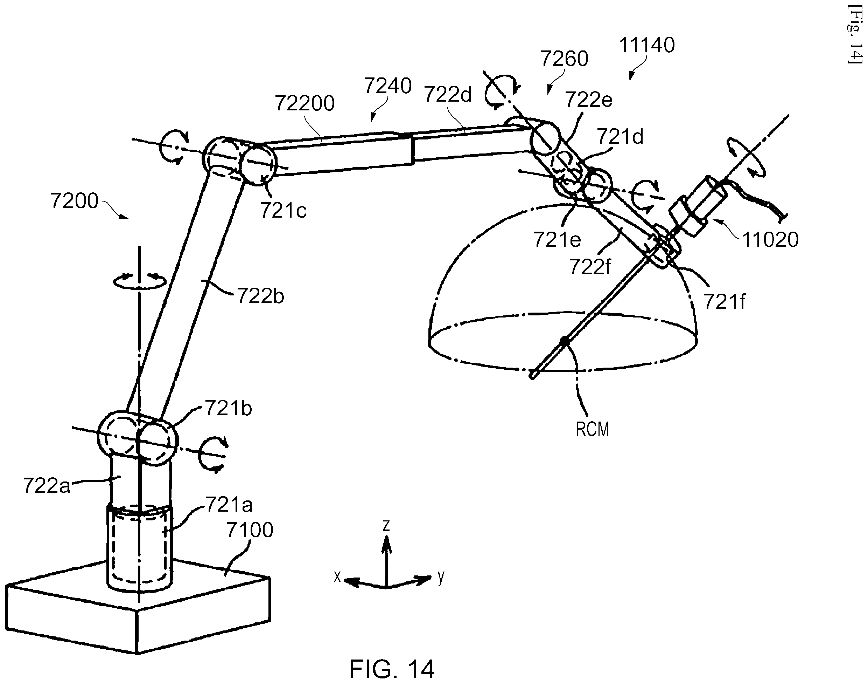

Fig. 14 shows an example of the arm unit 11140. The arm unit 12040 is configured in the same way. In this example, the arm unit 11140 supports an endoscope as an imaging device 11020. However, in another example, a different imaging device 11020 or surgical device 11030 (in the case of arm unit 11140) or 12080 (in the case of arm unit 12040) is supported.

The arm unit 11140 includes a base 7100 and an arm 7200 extending from the base 7100. The arm 7200 includes a plurality of active joints 721a to 721f and supports the endoscope 11020 at a distal end of the arm 7200. The links 722a to 722f are substantially rod-shaped members. Ends of the plurality of links 722a to 722f are connected to each other by active joints 721a to 721f, a passive slide mechanism 7240 and a passive joint 7260. The base unit 7100 acts as a fulcrum so that an arm shape extends from the base 7100.

A position and a posture of the endoscope 11020 are controlled by driving and controlling actuators provided in the active joints 721a to 721f of the arm 7200. According to this example, a distal end of the endoscope 11020 is caused to enter a patient's body cavity, which is a treatment site, and captures an image of the treatment site. However, the endoscope 11020 may instead be another device such as another imaging device or a surgical device. More generally, a device held at the end of the arm 7200 is referred to as a distal unit or distal device.

Here, the arm unit 7200 is described by defining coordinate axes as illustrated in Fig. 14 as follows. Furthermore, a vertical direction, a longitudinal direction, and a horizontal direction are defined according to the coordinate axes. In other words, a vertical direction with respect to the base 7100 installed on the floor surface is defined as a z-axis direction and the vertical direction. Furthermore, a direction orthogonal to the z axis, the direction in which the arm 7200 is extended from the base 7100 (in other words, a direction in which the endoscope 11020 is positioned with respect to the base 7100) is defined as a y-axis direction and the longitudinal direction. Moreover, a direction orthogonal to the y-axis and z-axis is defined as an x-axis direction and the horizontal direction.

The active joints 721a to 721f connect the links to each other to be rotatable. The active joints 721a to 721f have the actuators, and have each rotation mechanism that is driven to rotate about a predetermined rotation axis by drive of the actuator. As the rotational drive of each of the active joints 721a to 721f is controlled, it is possible to control the drive of the arm 7200, for example, to extend or contract (fold) the arm unit 7200.

The passive slide mechanism 7240 is an aspect of a passive form change mechanism, and connects the link 722c and the link 722d to each other to be movable forward and rearward along a predetermined direction. The passive slide mechanism 7240 is operated to move forward and rearward by, for example, a user, and a distance between the active joint 721c at one end side of the link 722c and the passive joint 7260 is variable. With the configuration, the whole form of the arm unit 7200 can be changed.

The passive joint 7360 is an aspect of the passive form change mechanism, and connects the link 722d and the link 722e to each other to be rotatable. The passive joint 7260 is operated to rotate by, for example, the user, and an angle formed between the link 722d and the link 722e is variable. With the configuration, the whole form of the arm unit 7200 can be changed.

In an embodiment, the arm unit 11140 has the six active joints 721a to 721f, and six degrees of freedom are realized regarding the drive of the arm 7200. That is, the passive slide mechanism 7260 and the passive joint 7260 are not objects to be subjected to the drive control while the drive control of the arm unit 11140 is realized by the drive control of the six active joints 721a to 721f.

Specifically, as illustrated in Fig. 14, the active joints 721a, 721d, and 721f are provided so as to have each long axis direction of the connected links 722a and 722e and a capturing direction of the connected endoscope 11020 as a rotational axis direction. The active joints 721b, 721c, and 721e are provided so as to have the x-axis direction, which is a direction in which a connection angle of each of the connected links 722a to 722c, 722e, and 722f and the endoscope 11020 is changed within a y-z plane (a plane defined by the y axis and the z axis), as a rotation axis direction. In this manner, the active joints 721a, 721d, and 721f have a function of performing so-called yawing, and the active joints 421b, 421c, and 421e have a function of performing so-called pitching.

Since the six degrees of freedom are realized with respect to the drive of the arm 7200 in the arm unit 11140 the endoscope 11020 can be freely moved within a movable range of the arm 7200. Fig. 14 illustrates a hemisphere as an example of the movable range of the endoscope 11020. Assuming that a central point RCM (remote center of motion) of the hemisphere is a capturing centre of a treatment site captured by the endoscope 11020, it is possible to capture the treatment site from various angles by moving the endoscope 11020 on a spherical surface of the hemisphere in a state where the capturing centre of the endoscope 11020 is fixed at the centre point of the hemisphere.

The invention is defined in the following claims.

## Claims

1. A system (800) for controlling a medical image capture device during surgery, the system including circuitry (810, 820, 830) configured to:
acquire first image data from the medical image capture device, the first image data being of an appearance of a surgical scene at a first instance of time;
determine, based on a predicted appearance of the surgical scene based on the first image data at a second instance of time after the first instance of time, one or more desired image capture properties of the medical image capture device; and
control the medical image capture device at a third instance of time, the third instance of time being between the first instance of time and the second instance of time, in accordance with the one or more desired image capture properties of the medical image capture device;
wherein the circuitry is configured to determine a desired imaging condition of the image capture device as one of the one or more desired image capture properties of the medical image capture device; wherein the desired imaging condition includes one or more of an optical image system condition and an image processing condition;
**characterised in that**
the optical image system condition and the image processing condition include at least one of an image zoom, an image focus, an image aperture, an image contrast and/or an image brightness of the medical image capture device.

2. The system according to Claim 1, wherein controlling the medical image capture device includes controlling the position of an articulated arm supporting the medical image capture device.

3. The system according to Claim 1, wherein the circuitry is configured to determine, based on the predicted appearance of the surgical scene, a desired location of the medical image capture device as one of the one or more desired image capture properties of the medical image capture device.

4. The system according to Claim 2, wherein the circuitry is further configured to determine a movement pattern to the desired image capture location in accordance with the location of one or more objects present in the scene, and to control the position and/or orientation of an articulated arm supporting the medical image capture device in accordance with the determined movement pattern.

5. The system according to Claim 1, wherein the circuitry is further configured to generate, in accordance with the first image data, second image data, the second image data being of the predicted appearance of the surgical scene at the second instance of time.

6. The system according to Claim 6, wherein the circuitry is further configured to generate the second image data in accordance with the first image data and information regarding the current status of the scene.

7. The system according to Claim 6, wherein the circuitry is further configured to acquire the information regarding the current status of the scene, the information regarding the current status of the scene including at least one of: the position of an object in the scene, the movement of an object in the scene, a type of object which is present in the scene and/or an action being performed by a person in the scene.

8. The system according to Claim 1, wherein the circuitry is configured to control the medical image capture device being one of an endoscope, a microscope, or an exoscope.

9. The system according to Claim 1, wherein, the circuitry is further configured to account for a limitation of the medical image capture device when determining the desired image capture properties of the medical image capture device.

10. The system according to Claim 1, wherein the circuitry is further configured to generate the predicted appearance of the surgical scene using a machine learning system trained on surgical data obtained in surgical scenarios.

11. The system according to Claim 10, wherein the surgical data obtained in surgical scenarios includes one or more of: images of past surgical scenarios, validated simulations of surgical scenarios and/or prior images of the present surgical scenario information regarding actions taken by a surgeon during previous surgical scenarios and/or image capture properties of a medical image capture device used during previous surgical scenarios.

12. The system according to Claim 1, wherein the circuitry is configured to calculate a weighting for image capture properties of the medical image capture device in accordance with one or more factors associated with those image capture properties, and determine the image capture properties having the highest weighting factor as the desired image capture properties for the medical image capture device.

13. The system according to Claim 1, wherein the circuitry is configured to calculate a range of movement which can be completed in the time between the third instance of time and the second instance of time, and determine the desired image capture properties of the medical image capture device in accordance with the calculation.

14. A method of controlling a medical image capture device during surgery,
the method including:
acquiring (1202) first image data from the medical image capture device, the first image data being of an appearance of a surgical scene at a first instance of time;
determining (1204), based on a predicted appearance of the surgical scene based on the first image data at a second instance of time after the first instance of time, one or more desired image capture properties of the medical image capture device; and
controlling (1206) the medical image capture device at a third instance of time, the third instance of time being between the first instance of time and the second instance of time, in accordance with the one or more desired image capture properties of the medical image capture device;
wherein the method comprises determining a desired imaging condition of the image capture device as one of the one or more desired image capture properties of the medical image capture device; wherein the desired imaging condition includes one or more of an optical image system condition and an image processing condition and wherein the optical image system condition and the image processing condition include at least one of an image zoom, an image focus, an image aperture, an image contrast and/or an image brightness of the medical image capture device.

15. A computer program product including instructions which, when the program is executed by a computer, cause the computer to carry out the method of controlling a medical image capture device according to claim 14.

## Patentansprüche

1. System (800) zum Steuern einer medizinischen Bildaufnahmevorrichtung während einer Operation, wobei das System eine Schaltlogik (810, 820, 830) einschließt, die konfiguriert ist, zum:
Erfassen erster Bilddaten von der medizinischen Bildaufnahmevorrichtung, wobei die ersten Bilddaten ein Erscheinungsbild einer Operationsszene zu einem ersten Zeitpunkt sind;
Bestimmen, basierend auf einem vorhergesagten Erscheinungsbild der Operationsszene basierend auf den ersten Bilddaten zu einem zweiten Zeitpunkt nach dem ersten Zeitpunkt, einer oder mehrerer gewünschter Bildaufnahmeeigenschaften der medizinischen Bildaufnahmevorrichtung; und
Steuern der medizinischen Bildaufnahmevorrichtung zu einem dritten Zeitpunkt, wobei der dritte Zeitpunkt zwischen dem ersten Zeitpunkt und dem zweiten Zeitpunkt liegt, gemäß der einen oder mehreren gewünschten Bildaufnahmeeigenschaften der medizinischen Bildaufnahmevorrichtung;
wobei die Schaltlogik konfiguriert ist, um eine gewünschte Bildgebungsbeschaffenheit der Bildaufnahmevorrichtung als eine der einen oder mehreren gewünschten Bildaufnahmeeigenschaften der medizinischen Bildaufnahmevorrichtung zu bestimmen; wobei die gewünschte Bildgebungsbeschaffenheit eine oder mehrere von einer optischen Bildsystembeschaffenheit und einer Bildverarbeitungsbeschaffenheit einschließt;
**dadurch gekennzeichnet, dass**
die optische Bildsystembeschaffenheit und die Bildverarbeitungsbeschaffenheit mindestens eines von einem Bildzoom, einem Bildfokus, einer Bildblende, einem Bildkontrast und/oder einer Bildhelligkeit der medizinischen Bildaufnahmevorrichtung einschließen.

2. System nach Anspruch 1, wobei das Steuern der medizinischen Bildaufnahmevorrichtung das Steuern der Position eines Gelenkarms einschließt, der die medizinische Bildaufnahmevorrichtung trägt.

3. System nach Anspruch 1, wobei die Schaltlogik konfiguriert ist, um, basierend auf dem vorhergesagten Erscheinungsbild der Operationsszene, einen gewünschten Ort der medizinischen Bildaufnahmevorrichtung als eine der einen oder mehreren gewünschten Bildaufnahmeeigenschaften der Bildaufnahmevorrichtung zu bestimmen.

4. System nach Anspruch 2, wobei die Schaltlogik ferner konfiguriert ist, um ein Bewegungsmuster zu dem gewünschten Bildaufnahmeort gemäß des Orts von einem oder mehreren Objekten, die in der Szene vorhanden sind, zu bestimmen, und um die Position und/oder Ausrichtung eines Gelenkarms, der die medizinische Bildaufnahmevorrichtung trägt, gemäß dem bestimmten Bewegungsmuster zu steuern.

5. System nach Anspruch 1, wobei die Schaltlogik ferner konfiguriert ist, um gemäß den ersten Bilddaten zweite Bilddaten zu erzeugen, wobei die zweiten Bilddaten das vorhergesagte Erscheinungsbild der Operationsszene zu dem zweiten Zeitpunkt sind.

6. System nach Anspruch 6, wobei die Schaltlogik ferner konfiguriert ist, um die zweiten Bilddaten gemäß den ersten Bilddaten und Informationen bezüglich des aktuellen Status der Szene zu erzeugen.

7. System nach Anspruch 6, wobei die Schaltlogik ferner konfiguriert ist, um die Informationen bezüglich des aktuellen Status der Szene zu erfassen, wobei die Informationen bezüglich des aktuellen Status der Szene mindestens eines von der Position eines Objekts in der Szene, der Bewegung eines Objekts in der Szene, eines Typs eines in der Szene vorhandenen Objekts und/oder einer durch eine Person in der Szene durchgeführte Maßnahme einschließen.

8. System nach Anspruch 1, wobei die Schaltlogik konfiguriert ist, um die medizinische Bildaufnahmevorrichtung, die eines von einem Endoskop, einem Mikroskop oder einem Exoskop ist, zu steuern.

9. System nach Anspruch 1, wobei die Schaltlogik ferner konfiguriert ist, um eine Einschränkung der medizinischen Bildaufnahmevorrichtung zu berücksichtigen, wenn die gewünschten Bildaufnahmeeigenschaften der medizinischen Bildaufnahmevorrichtung bestimmt werden.

10. System nach Anspruch 1, wobei die Schaltlogik ferner konfiguriert ist, um das vorhergesagte Erscheinungsbild der Operationsszene unter Verwendung eines Maschinenlernsystems zu erzeugen, das mit Operationsdaten trainiert wird, die in Operationsszenarien erhalten werden.

11. System nach Anspruch 10, wobei die Operationsdaten, die in Operationsszenarien erhalten werden, eines oder mehrere einschließen von: Bildern früherer Operationsszenarien, validierten Simulationen von Operationsszenarien und/oder früheren Bildern der aktuellen Operationsszenarioinformationen bezüglich der durch einen Chirurgen während früherer Operationsszenarien ergriffener Maßnahmen und/oder Bildaufnahmeeigenschaften einer während früherer Operationsszenarien verwendeten medizinischen Bildaufnahmevorrichtung.

12. System nach Anspruch 1, wobei die Schaltlogik konfiguriert ist, um eine Gewichtung für Bildaufnahmeeigenschaften der medizinischen Bildaufnahmevorrichtung gemäß einem oder mehreren Faktoren zu berechnen, die mit diesen Bildaufnahmeeigenschaften verknüpft sind, und die Bildaufnahmeeigenschaften, die den höchsten Gewichtungsfaktor aufweisen, als die gewünschten Bildaufnahmeeigenschaften für die medizinische Bildaufnahmevorrichtung zu bestimmen.

13. System nach Anspruch 1, wobei die Schaltlogik konfiguriert ist, um einen Bewegungsbereich zu berechnen, der in der Zeit zwischen dem dritten Zeitpunkt und dem zweiten Zeitpunkt abgeschlossen werden kann, und die gewünschten Bildaufnahmeeigenschaften der medizinischen Bildaufnahmevorrichtung in gemäß der Berechnung zu bestimmen.

14. Verfahren zum Steuern einer medizinischen Bildaufnahmevorrichtung während einer Operation, wobei das Verfahren einschließt:
Erfassen (1202) erster Bilddaten von der medizinischen Bildaufnahmevorrichtung, wobei die ersten Bilddaten ein Erscheinungsbild einer Operationsszene zu einem ersten Zeitpunkt sind;
Bestimmen (1204), basierend auf einem vorhergesagten Erscheinungsbild der Operationsszene basierend auf den ersten Bilddaten zu einem zweiten Zeitpunkt nach dem ersten Zeitpunkt, einer oder mehrerer gewünschter Bildaufnahmeeigenschaften der medizinischen Bildaufnahmevorrichtung; und
Steuern (1206) der medizinischen Bildaufnahmevorrichtung zu einem dritten Zeitpunkt, wobei der dritte Zeitpunkt zwischen dem ersten Zeitpunkt und dem zweiten Zeitpunkt liegt, gemäß der einen oder mehreren gewünschten Bildaufnahmeeigenschaften der medizinischen Bildaufnahmevorrichtung;
wobei das Verfahren das Bestimmen einer gewünschten Bildgebungsbeschaffenheit der Bildaufnahmevorrichtung als eine der einen oder mehreren gewünschten Bildaufnahmeeigenschaften der medizinischen Bildaufnahmevorrichtung umfasst; wobei die gewünschte Bildgebungsbeschaffenheit eine oder mehrere von einer optischen Bildsystembeschaffenheit und einer Bildverarbeitungsbeschaffenheit einschließt, und wobei die optische Bildsystembeschaffenheit und die Bildverarbeitungsbeschaffenheit mindestens eines von einem Bildzoom, einem Bildfokus, einer Bildblende, einem Bildkontrast und/oder einer Bildhelligkeit der medizinischen Bildaufnahmevorrichtung einschließen.

15. Computerprogrammprodukt, das Anweisungen einschließt, die, wenn das Programm durch einen Computer ausgeführt wird, den Computer veranlassen, das Verfahren zum Steuern eine medizinischen Bildaufnahmevorrichtung nach Anspruch 14 vorzunehmen.

## Revendications

1. Système (800) permettant de commander un dispositif de capture d'image médicale au cours d'une opération chirurgicale, le système comportant une circuiterie (810, 820, 830) configurée pour :
acquérir des premières données d'image à partir du dispositif de capture d'image médicale, les premières données d'image représentant une apparence d'une scène chirurgicale à une première instance de temps ;
déterminer, sur la base d'une apparence prédite de la scène chirurgicale sur la base des premières données d'image à une deuxième instance de temps après la première instance de temps, une ou plusieurs propriétés de capture d'image souhaitées du dispositif de capture d'image médicale ; et
commander le dispositif de capture d'image médicale à une troisième instance de temps, la troisième instance de temps se situant entre la première instance de temps et la deuxième instance de temps, conformément à la ou aux propriétés de capture d'image souhaitées du dispositif de capture d'image médicale ;
dans lequel la circuiterie est configurée pour déterminer une condition d'imagerie souhaitée du dispositif de capture d'image comme l'une de la ou des propriétés de capture d'image souhaitées du dispositif de capture d'image médicale ; dans lequel la condition d'imagerie souhaitée comporte un ou plusieurs parmi une condition de système d'image optique et une condition de traitement d'image ;
**caractérisé en ce que**
la condition de système d'image optique et la condition de traitement d'image comportent au moins l'un parmi un zoom d'image, une mise au point d'image, une ouverture d'image, un contraste d'image et/ou une luminosité d'image du dispositif de capture d'image médicale.

2. Système selon la revendication 1, dans lequel la commande du dispositif de capture d'image médicale comporte la commande de la position d'un bras articulé supportant le dispositif de capture d'image médicale.

3. Système selon la revendication 1, dans lequel la circuiterie est configurée pour déterminer, sur la base de l'apparence prédite de la scène chirurgicale, un emplacement souhaité du dispositif de capture d'image médicale comme l'une de la ou des propriétés de capture d'image souhaitée du dispositif de capture d'image médicale.

4. Système selon la revendication 2, dans lequel la circuiterie est en outre configurée pour déterminer un modèle de déplacement vers l'emplacement de capture d'image souhaité conformément à l'emplacement d'un ou de plusieurs objets présents dans la scène, et pour commander la position et/ou l'orientation d'un bras articulé supportant le dispositif de capture d'image médicale conformément au modèle de déplacement déterminé.

5. Système selon la revendication 1, dans lequel la circuiterie est en outre configurée pour générer, conformément aux premières données d'image, des secondes données d'image, les secondes données d'image représentant l'apparence prédite de la scène chirurgicale à la deuxième instance de temps.

6. Système selon la revendication 6, dans lequel la circuiterie est en outre configurée pour générer les secondes données d'image conformément aux premières données d'image et à des informations relatives à l'état actuel de la scène.

7. Système selon la revendication 6, dans lequel la circuiterie est en outre configurée pour acquérir les informations relatives à l'état actuel de la scène, les informations relatives à l'état actuel de la scène comportant au moins l'un parmi la position d'un objet dans la scène, le déplacement d'un objet dans la scène, un type d'objet présent dans la scène et/ou une action réalisée par une personne dans la scène.

8. Système selon la revendication 1, dans lequel la circuiterie est configurée pour commander le dispositif de capture d'image médicale comme un endoscope, un microscope ou un exoscope.

9. Système selon la revendication 1, dans lequel la circuiterie est en outre configurée pour constituer une limitation du dispositif de capture d'image médicale lors de la détermination des propriétés de capture d'image souhaitées du dispositif de capture d'image médicale.

10. Système selon la revendication 1, dans lequel la circuiterie est en outre configurée pour générer l'apparence prédite de la scène chirurgicale à l'aide d'un système d'apprentissage automatique entraîné sur des données chirurgicales obtenues dans des scénarios chirurgicaux.

11. Système selon la revendication 10, dans lequel les données chirurgicales obtenues dans des scénarios chirurgicaux comportent un ou plusieurs parmi : des images de scénarios chirurgicaux antérieurs, des simulations validées de scénarios chirurgicaux et/ou des images antérieures du scénario chirurgical présent, des informations relatives à des actions prises par un chirurgien au cours des scénarios chirurgicaux antérieurs et/ou des propriétés de capture d'image d'un dispositif de capture d'image médicale utilisé au cours des scénarios chirurgicaux antérieurs.

12. Système selon la revendication 1, dans lequel la circuiterie est configurée pour calculer une pondération pour des propriétés de capture d'image du dispositif de capture d'image médicale conformément à un ou plusieurs facteurs associés à ces propriétés de capture d'image, et déterminer les propriétés de capture d'image présentant le facteur de pondération le plus élevé comme étant les propriétés de capture d'image souhaitées pour le dispositif de capture d'image médicale.

13. Système selon la revendication 1, dans lequel la circuiterie est configurée pour calculer une plage de déplacements qui peuvent être accomplis dans le temps entre la troisième instance de temps et la deuxième instance de temps, et déterminer les propriétés de capture d'image souhaitées du dispositif de capture d'image médicale conformément au calcul.

14. Procédé permettant de commander un dispositif de capture d'image médicale au cours d'une opération chirurgicale, le procédé comportant :
l'acquisition (1202) de premières données d'image à partir du dispositif de capture d'image médicale, les premières données d'image représentant une apparence d'une scène chirurgicale à une première instance de temps ;
la détermination (1204), sur la base d'une apparence prédite de la scène chirurgicale basée sur les premières données d'image à une deuxième instance de temps après la première instance de temps, d'une ou de plusieurs propriétés de capture d'image souhaitées du dispositif de capture d'image médicale ; et
la commande (1206) du dispositif de capture d'image médicale à une troisième instance de temps, la troisième instance de temps se situant entre la première instance de temps et la deuxième instance de temps, conformément à la ou aux propriétés de capture d'image souhaitées du dispositif de capture d'image médicale ;
dans lequel le procédé comprend la détermination d'une condition d'imagerie souhaitée du dispositif de capture d'image comme l'une des propriétés de capture d'image souhaitées du dispositif de capture d'image médicale ; dans lequel la condition d'imagerie souhaitée comporte un ou plusieurs parmi une condition de système d'image optique et une condition de traitement d'image, et dans lequel la condition de système d'image optique et la condition de traitement d'image comportent au moins l'un parmi un zoom d'image, une mise au point d'image, une ouverture d'image, un contraste d'image et/ou une luminosité d'image du dispositif de capture d'image médicale.

15. Produit-programme informatique comportant des instructions qui, lorsque le programme est exécuté par un ordinateur, amènent l'ordinateur à mettre en œuvre le procédé permettant de commander un dispositif de capture d'image médicale selon la revendication 14.
